# EUROPEAN PATENT SPECIFICATION

(11) **EP 1 451 161 B1**
(45) Date of publication and mention of the grant of the patent: **18.04.2007**
(21) Application number: 02779772.9
(22) Date of filing: 27.11.2002
(51) Int. Cl.: C07D 235/04, C07D 487/04, A61K 31/4184, A61K 31/53, A61P 25/00

(54) **IMIDAZOPYRIDINES, PYRIMIDINES AND TRIAZINES FOR ENHANCING COGNITION AS GABA-A ALPHA 5 RECEPTOR SUBTYPE LIGANDS**
IMIDAZOPYRIDINE, -PYRIMIDINE UND -TRIAZINE ALS GABA-A ALPHA 5 REZEPTOR SUBTYP LIGANDEN ZUR VERBESSERUNG KOGNITIVER EIGENSCHAFTEN
IMIDAZOPYRIDINES, PYRIMIDINES ET TRIAZINES ACTIVATEURS DE LA COGNITION UTILISES COMME LIGANDS DE LA SOUS-UNITE ALPHA 5 DU RECEPTEUR GABA-A

(30) Priority: 28.11.2001 GB 0128499
(43) Date of publication of application: 01.09.2004
(73) Proprietor: Merck Sharp & Dohme Limited, Hoddesdon, Hertfordshire EN11 9BU (GB)
(72) Inventor: BETTATI, Michela, Harlow, Essex CM20 2QR (GB); CHAMBERS, Mark Stuart, Harlow, Essex CM20 2QR (GB); HUMPHRIES, Alexander Charles, Harlow, Essex CM20 2QR (GB); JONES, Philip IRBM, I-00040 Pomezia (Rome) (IT); LEWIS, Richard Thomas, Harlow, Essex CM20 2QR (GB); MAXEY, Robert James, Harlow, Essex CM20 2QR (GB); SZEKERES, Helen Jane, Harlow, Essex CM20 2QR (GB); TEALL, Martin Richard, Harlow, Essex CM20 2QR (GB)
(74) Representative: Horgan, James Michael Frederic
(86) International application number: PCT/GB2002/005337
(87) International publication number: WO 2003/048132

(56) References cited:
- WO-A-01/38326
- WO-A-01/90108
- WO-A-02/38568
- WO-A-02/074772
- WO-A-02/074773
- LIEBSCHER J ET AL: "SYNTHESIS OF IMIDAZOLES BY REACTION OF N-BENZYLATED AMIDINES WITH CARBOXYLIC ACID DERIVATIVES" JOURNAL FUER PRAKTISCHE CHEMIE, LEIPZIG, DE, vol. 2, no. 330, 1988, pages 175-181, XP001069598 ISSN: 0021-8383

## Description

The present invention relates to a class of substituted fused imidazole derivatives and to their use in therapy. More particularly, this invention is concerned with substituted imidazo[1,2-*a*]pyrimidine, imidazo[1,2-*b*][1,2,4]triazine and imidazo[1,2-*a*]pyridine derivatives which are ligands for GABA_{A} receptors containing the α5 subunit and are therefore useful in therapy where cognition enhancement is required.

Receptors for the major inhibitory neurotransmitter, gamma-aminobutyric acid (GABA), are divided into two main classes: (1) GABA_{A} receptors, which are members of the ligand-gated ion channel superfamily; and (2) GABA_{B} receptors, which may be members of the G-protein linked receptor superfamily. Since the first cDNAs encoding individual GABA_{A} receptor subunits were cloned, the number of known members of the mammalian family has grown to include at least six α subunits, four β subunits, three γ subunits, one δ subunit, one ε subunit and two ρ subunits.

Although knowledge of the diversity of the GABA_{A} receptor gene family represents a huge step forward in our understanding of this ligand-gated ion channel, insight into the extent of subtype diversity is still at an early stage. It has been indicated that an α subunit, a β subunit and a γ subunit constitute the minimum requirement for forming a fully functional GABA_{A} receptor expressed by transiently transfecting cDNAs into cells. As indicated above, δ, c and ρ subunits also exist, but are present only to a minor extent in GABA_{A} receptor populations.

Studies of receptor size and visualisation by electron microscopy conclude that, like other members of the ligand-gated ion channel family, the native GABA_{A} receptor exists in pentameric form. The selection of at least one α, one β and one γ subunit from a repertoire of seventeen allows for the possible existence of more than 10,000 pentameric subunit combinations. Moreover, this calculation overlooks the additional permutations that would be possible if the arrangement of subunits around the ion channel had no constraints (i.e. there could be 120 possible variants for a receptor composed of five different subunits).

Receptor subtype assemblies which do exist include α1β2γ2, α2β2/3γ2, α3βγ2/3, α2βγ1, α5β3γ2/3, α6βγ2, α6βδ and α4βδ. Subtype assemblies containing an α1 subunit are present in most areas of the brain and account for over 40% of GABA_{A} receptors in the rat. Subtype assemblies containing α2 and α3 subunits respectively account for about 25% and 17% of GABA_{A} receptors in the rat. Subtype assemblies containing an α5 subunit are expressed predominantly in the hippocampus and cortex and are thought to represent about 4% of receptors in the rat.

A characteristic property of all known GABA_{A} receptors is the presence of a number of modulatory sites, one of which is the benzodiazepine (BZ) binding site. The BZ binding site is the most explored of the GABA_{A} receptor modulatory sites, and is the site through which anxiolytic drugs such as diazepam and temazepam exert their effect. Before the cloning of the GABA_{A} receptor gene family, the benzodiazepine binding site was historically subdivided into two subtypes, BZ1 and BZ2, on the basis of radioligand binding studies. The BZ1 subtype has been shown to be pharmacologically equivalent to a GABA_{A} receptor comprising the α1 subunit in combination with β2 and γ2. This is the most abundant GABA_{A} receptor subtype, representing almost half of all GABA_{A} receptors in the brain.

A number of dementing illnesses such as Alzheimer's disease are characterised by a progressive deterioration in cognition in the sufferer. It would clearly be desirable to enhance cognition in subjects desirous of such treatment, for example for subjects suffering from a dementing illness.

It has been reported by McNamara and Skelton in Psychobiology, 21:101-108, that the benzodiazepine receptor inverse agonist β-CCM enhanced spatial learning in the Morris watermaze. However, β-CCM and other conventional benzodiazepine receptor inverse agonists are proconvulsant which makes it clear that they cannot be used as cognition enhancing agents in humans.

However, we have now discovered that it is possible to obtain medicaments which have cognition enhancing effects which may be employed with less risk of proconvulsant effects previously described with benzodiazepine receptor partial or full inverse agonists.

It has now been discovered that use of an α5 receptor partial or full inverse agonist which is relatively free of activity at α1 and/or α2 and/or α3 receptor binding sites can be used to provide a medicament which is useful for enhancing cognition but in which proconvulsant activity is reduced or eliminated. Inverse agonists at α5 which are not free of activity at α1 and/or α2 and/or α3 but which are functionally selective for α5 can also be used. Inverse agonists which are both selective for α5 and are relatively free of activity at α1, α2 and α3 receptor binding sites are preferred.

International Patent. Publication No. WO01/038326 discloses certain 3-phenylimidazo[1,2-*a*]pyridines to be ligands for GABA_{A} receptors, but neither discloses nor suggests compounds in accordance with the present invention

According to the invention there is provided a pharmaceutical composition comprising, in a pharmaceutically acceptable carrier, one or more compounds of formula I or pharmaceutically-acceptable salts thereof: 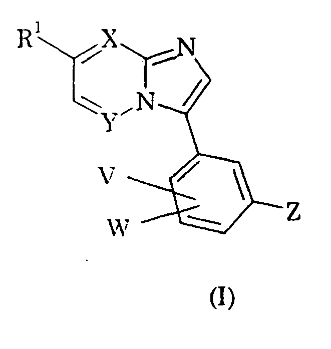 wherein:
X represents N and Y represents N or CH;
R¹ represents hydrogen, hydrocarbon, a heterocyclic group, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NRₐCO₂R^{b}, -COR^{a}, -CO₂R^{a}, -CONR^{a}R^{b} or -CR^{a}=NOR^{b};
R^{a} and R^{b} independently represent hydrogen, hydrocarbon or a heterocyclic group;
V and W are independently selected from H, halogen, C₁₋₆alkyl, OH and C₁₋₆alkoxy;
Z represents H, halogen, CN, NO₂, CF₃, OCF₃, CF₂H, SCF₃, R², OR³, SR³, (CH₂)ₚN(R³)₂, O(CH₂)ₚN(R³)₂, SO₂R², SO₂N(R³)₂, COR⁴, CO₂R³, CON(R³)₂, NHCOR⁴, NR'(CH₂)ₙheteroaryl or O(CH₂)ₙheteroaryl where the heteroaryl is optionally substituted by one, two or three groups chosen from C₁₋₆alkyl, benzyl, (CH₂)ₚN(R³)₂, halogen and CF₃, R' is C₁₋₆alkyl, n is 1 or 2 and p is 0, 1 or 2;
with the proviso that at least one of V, W and Z is other than H;
R² represents C₁₋₆alkyl, C₃₋₆cycloalkyl, C₃₋₆cycloalkylC₁₋₄alkyl, C₂₋₆alkenyl or C₂₋₈alkynyl, any of which may bear a substituent selected from halogen, CN, NO₂, CF₃, OCF₃, CF₂H, SCF₃, OH, C₁₋₄alkoxy, C₁₋₄alkoxycarbonyl, amino, C₁₋₄alkylamino, or di(C₁₋₄alkyl)amino;
R³ represents H or R²; or two R³ groups bonded to the same nitrogen atom may complete a 5-7 membered nonaromatic heterocyclic ring; and
R⁴ represents R³ or heteroaryL

In a second aspect, the invention provides a compound of formula I as defined above, or a pharmaceutically acceptable salt thereof, for use in treatment of the human body. Preferably the treatment is for a condition associated with GABA_{A} receptors comprising the α5 subunit and/or for the enhancement of cognition. Preferably the condition is a neurological deficit with an associated cognitive disorder such as a dementing illness such as Alzheimer's disease. Other conditions to be treated include cognition deficits due to traumatic injury, stroke, Parkinson's disease, Downs syndrome, age related memory deficits, attention deficit disorder and the like.

The present invention further provides the use of a compound of formula I as defined above, or a pharmaceutically acceptable salt thereof, in the manufacture of a medicament for the enhancement of cognition, preferably in a human suffering from a dementing illness such as Alzheimer's disease.

Also disclosed is a method of treatment of a subject suffering from a cognition deficit, such as that resulting from a dementing illness such as Alzheimer's disease, which comprises administering to that subject an effective amount of a compound according to the present invention.

In a further aspect, the present invention provides a compound of formula I as defined above, or a pharmaceutically acceptable salt thereof, with the proviso that Z is other than halogen, OH, OCH₃ or NH₂.

For use in medicine, the salts of the compounds of formula I will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

The term "hydrocarbon" as used herein includes straight-chained, branched and cyclic groups containing up to 18 carbon atoms, suitably up to 15 carbon atoms, and conveniently up to 12 carbon atoms. Suitable hydrocarbon groups include C₁₋₆ alkyl, C₂₋₆ alkenyl, C₂₋₆ alkynyl, C₃₋₇ cycloalkyl, C₃₋₇ cycloalkyl(C₁₋₆)alkyl, indanyl, aryl and aryl(C₁₋₆)alkyl.

The expression "a heterocyclic group" as used herein includes cyclic groups containing up to 18 carbon atoms and at least one heteroatom preferably selected from oxygen, nitrogen and sulphur. The heterocyclic group suitably contains up to 15 carbon atoms and conveniently up to 12 carbon atoms, and is preferably linked through carbon. Examples of suitable heterocyclic groups include C₃₋₇ heterocycloalkyl, C₃₋₇ heterocycloalkyl(C₁₋₆)alkyl, heteroaryl and heteroaryl(C₁₋₆)alkyl groups.

Suitable alkyl groups include straight-chained and branched alkyl groups containing from 1 to 6 carbon atoms. Typical examples include methyl and ethyl groups, and straight-chained or branched propyl, butyl and pentyl groups. Particular alkyl groups are methyl, ethyl, *n*-propyl, isopropyl, isobutyl, *tert*-butyl and 2,2-dimethylpropyl. Derived expressions such as "C₁₋₆ alkoxy", "C₁₋₆ alkylamino" and "C₁₋₆ alkylsulphonyl" are to be construed accordingly.

Suitable alkenyl groups include straight-chained and branched alkenyl groups containing from 2 to 6 carbon atoms. Typical examples include vinyl, allyl and dimethylallyl groups.

Suitable alkynyl groups include straight-chained and branched alkynyl groups containing from 2 to 6 carbon atoms. Typical examples include ethynyl and propargyl groups.

Suitable cycloalkyl groups include groups containing from 3 to 7 carbon atoms. Particular cycloalkyl groups are cyclopropyl and cyclohexyl.

Typical examples of C₃₋₇ cycloalkyl(C₁₋₆)alkyl groups include cyclopropylmethyl, cyclohexylmethyl and cyclohexylethyl.

Particular indanyl groups include indan-1-yl and indan-2-yl.

Particular aryl groups include phenyl and naphthyl, preferably phenyl.

Particular aryl(C₁₋₆)alkyl groups include benzyl, phenylethyl, phenylpropyl and naphthylmethyl.

Suitable heterocycloalkyl groups include azetidinyl, pyrrolidinyl, piperidinyl, piperazinyl, morpholinyl and thiomorpholinyl groups.

A typical C₃₋₇ heterocycloalkyl(C₁₋₆)alkyl group is morpholinylmethyl.

Suitable heteroaryl groups include pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl and tetrazolyl groups.

The expression "heteroaryl(C₁₋₆)alkyl" as used herein includes furylmethyl, furylethyl, thienylmethyl, thienylethyl, oxazolylmethyl, oxazolylethyl, thiazolylmethyl, thiazolylethyl, imidazolylmethyl, imidazolylethyl, pyrazolylmethyl, pyrazolylethyl, oxadiazolylmethyl, oxadiazolylethyl, thiadiazolylmethyl, thiadiazolylethyl, triazolylmethyl, triazolylethyl, tetrazolylmethyl, tetrazolylethyl, pyridinylmethyl, pyridinylethyl, pyrimidinylmethyl, pyrazinylmethyl, quinolinylmethyl and isoquinolinylmethyl.

The hydrocarbon and heterocyclic groups may in turn be optionally substituted by one or more groups selected from C₁₋₆ alkyl, adamantyl, phenyl, halogen, C₁₋₆haloalkyl, C₁₋₆aminoalkyl, trifluoromethyl, hydroxy, C₁₋₆alkoxy, aryloxy, keto, C₁₋₃alkylenedioxy, nitro, cyano, carboxy, C₁₋₆alkoxycarbonyl, C₁₋₆alkoxycarbonyl(C₁₋₆)alkyl, C₁₋₆alkylcarbonyloxy, arylcarbonyloxy, aminocarbonyloxy, C₁₋₆alkylcarbonyl, arylcarbonyl, C₁₋₆ alkylthio, C₁₋₆ alkylsulphinyl, C₁₋₆ alkylsulphonyl, arylsulphonyl, -NR^{v}R^{w}, -NR^{v}COR^{w}, -NR^{v}CO₂R^{w}, -NR^{v}SO₂R^{w}, -CH₂NR^{v}SO₂R^{w}, -NHCONR^{v}R^{w}, -CONR^{v}R^{w}, -SO₂NR^{v}R^{w} and -CH₂SO₂NR^{v}R^{w}, in which R^{v} and R^{w} independently represent hydrogen, C₁₋₆alkyl, aryl or aryl(C₁₋₆)alkyl.

The term "halogen" as used herein includes fluorine, chlorine, bromine and iodine, especially fluoro or chloro.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

Typically, R¹ represents hydrogen, hydrocarbon, a heterocyclic group, halogen, cyano, trifluoromethyl, -OR^{a}, -COR^{a}, -CO₂R^{a}, -CONR^{a}R^{b} or -CR^{a}=NOR^{b}. Suitably, R¹ represents hydrocarbon, a heterocyclic group, cyano, halogen, trifluoromethyl, -OR^{a}, -COR^{a}, -CO₂R^{a}, -CONR^{a}R^{b} or -CR^{a}=NOR^{b}.

Typical values of R^{a} include hydrogen and C₁₋₆ alkyl. Suitably, R^{a} represents hydrogen or methyl.

Typical values of R^{b} include hydrogen, C₁₋₆ alkyl, hydroxy(C₁₋₆)alkyl and di(C₁₋₆)alkylamino(C₁₋₆)alkyl. Suitably, R^{b} represents hydrogen, methyl, ethyl, hydroxyethyl or dimethylaminoethyl. Particular values of R^{b} include hydrogen, hydroxyethyl and dimethylaminoethyl, especially hydrogen or dimethylaminoethyl.

Representative values of R¹ include hydrogen, C₁₋₆ alkyl, halo(C₁₋₆)alkyl, dihalo(C₁₋₆)alkyl, hydroxy(C₁₋₆)alkyl, C₁₋₆ alkoxy(C₁₋₆)alkyl, di(C₁₋₆)alkoxy(C₁₋₆)alkyl, C₃₋₇ cycloalkyl, C₃₋₇ heterocycloalkyl(C₁₋₆)alkyl, heteroaryl, C₁₋₆ alkyl-heteroaryl, heteroaryl(C₁₋₆)alkyl, halogen, cyano, trifluoromethyl, C₁₋₆ alkoxy, formyl, C₁₋₆alkylcarbonyl, C₁₋₆alkoxycarbonyl, -CONR^{a}R^{b} and -CR^{a}=NOR^{b}, in which R^{a} and R^{b} are as defined above.

Illustrative values of R¹ include C₁₋₆ alkyl, hydroxy(C₁₋₆)alkyl, heteroaryl, halogen, cyano, trifluoromethyl, C₁₋₆ alkoxy, formyl, C₁₋₆alkylcarbonyl, C₁₋₆alkoxycarbonyl, -CO₂H, -CONR^{a}R^{b} and -CRₐ=NOR^{b}, in which R^{a} and R^{b} are as defined above.

Itemised values of R¹ include hydrogen, methyl, fluoromethyl, difluoromethyl, hydroxymethyl, methoxymethyl, dimethoxymethyl, hydroxyethyl (especially 1-hydroxyethyl), fluoroethyl (especially 1-fluoroethyl), difluoroethyl (especially 1,1-difluoroethyl), dimethoxyethyl (especially 1,1-dimethoxyethyl), isopropyl, hydroxypropyl (especially 2-hydroxyprop-2-yl), fluoropropyl (especially 2-fluoroprop-2-yl), *tert*-butyl, cyclopropyl, cyclobutyl, morpholinylmethyl, pyridinyl, furyl, thienyl, oxazolyl, isoxazolyl, methylisoxazolyl, methylthiazolyl, methyloxadiazolyl, imidazolylmethyl, triazolylmethyl, chloro, cyano, trifluoromethyl, methoxy, formyl, acetyl, methoxycarbonyl, -CO₂H, carbamoyl and -CR^{aa}=NOR^{bb}, in which R^{aa} represents hydrogen or methyl, and R^{bb} represents hydrogen, hydroxyethyl or dimethylaminoethyl.

Selected values of R¹ include hydrogen, methyl, fluoromethyl, difluoromethyl, hydroxymethyl, dimethoxymethyl, dimethoxyethyl (especially 1,1-dimethoxyethyl), isopropyl, hydroxypropyl (especially 2-hydroxyprop-2-yl), *tert*-butyl, cyclopropyl, cyclobutyl, morpholinylmethyl, methylisoxazolyl, cyano, chloro, trifluoromethyl, methoxy, formyl, acetyl, methoxycarbonyl, carbamoyl, -CO₂H and -CR^{aa}=NOR^{bb}, in which R^{aa} and R^{bb} are as defined above.

Specific values of R¹ include cyano, chloro, trifluoromethyl, formyl, -CO₂H, methoxycarbonyl, carbamoyl, -CH=NOH and 5-methylisoxazol-3-yl.

In one favoured embodiment, R¹ represents trifluoromethyl.

In a particular embodiment, V, Z and W occupy the 2-, 3- and 4-positions respectively of the phenyl ring.

Typically, V and W independently represent H, halogen (especially Cl or F) or C₁₋₆alkyl (such as methyl, ethyl, n-propyl or isopropyl, especially methyl). In a particular embodiment, V represents H, Cl or F and W represents H, Cl, F or methyl, and typically at least one of V and W represents H.

In one embodiment Z represents H, halogen, CN, NO₂, CF₃, OCF₃, CF₂H, SCF₃, R², OR³, SR³, N(R³)₂, SO₂R², SO₂N(R³)₂, COR₄, CO₂R³, CON(R³)₂, NHCOR⁴, NH(CH₂)ₙheteroaryl or O(CH₂)ₙheteroaryl where n is 1 or 2.

Z represents H, halogen, CN, NO₂, CF₃, OCF₃, CF₂H, SCF₃, R², OR³, SR³, N(R³)₂, SO₂R², SO₂N(R³)₂, COR⁴, CO₂R³, CON(R³)₂, NHCOR⁴, NH(CH₂)ₙheteroaryl or O(CH₂)ₙheteroaryl where n is 1 or 2, where R², R³ and R⁴ are as previously defined.

At least one of V, Z and W is other than H.

R2 preferably represents C₁₋₆alkyl, such as methyl, ethyl, n-propyl, isopropyl, n-butyl or t-butyl, optionally substituted as defined previously. Typical substituents include halogen, hydroxy and methoxy. Preferably, alkyl groups represented by R² are unsubstituted or substituted with methoxy.

R³ represents H or R², or two R³ groups bonded to the same nitrogen atom can complete a 5-7 membered nonaromatic heterocycle. Examples of such N(R³)₂ groups include pyrrolidin-1-yl, piparidin-1-yl, piperazin-1-yl, morpholin-4-yl and thiomorpholin-4-yl.

R⁴ represents R³ or heteroaryl. Examples of heteroaryl groups represented by R⁴ include pyridyl, especially 3-pyridyl.

When Z represents -NH(CH₂)ₙheteroaryl or -O(CH₂)ₙheteroaryl, n is preferably 1. and the heteroaryl moiety is preferably pyridyl, especially 3-pyridyl, or is a 5-membered ring, such as pyrazole, imidazole, triazole and substituted derivatives thereof. Preferred substituents include alkyl (especially methyl) and trifluoromethyl.

Examples of groups represented by Z therefore include H, halogen (especially Br, Cl and F), CN, NO₂, CF₃, OCF₃, CF₂H, SCF₃, amino, t-butylamino, dimethylamino, morpholin-4-yl, methyl, methoxymethyl, isopropyl, methoxy, methylthio, acetamido, N,N-dimethylcarbamoyl, methanesulphonyl, N,N-dimethylaminosulphonyl, 3-pyridylcarbonyl, (3-pyridylmethyl)amino, (1,5-dimethylpyrazol-4-ylmethyl)amino, (1-methyl-5-trifluoromethylpyrazol-4-ylmethyl)amino, (2-methyl-[1,2,4]triazol-3-yl)methoxy, (1-methyl-[1,2,4]triazol-3-yl)methoxy and (1,5-dimethylpyrazol-4-yl)methoxy. Further specific examples of Z include CHO, morpholin-4-ylmethyl, N-t-butylaminomethyl, acetyl, N,N-dimethylaminomethyl, pyrid-2-ylmethoxy, pyrid-3-ylmethoxy, 1-methyl-[1,2,3,]triazol-4-ylmethoxy, pyrid-4-ylmethoxy, 5-t-butyl-[1,2,4]oxadiazol-3-ylmethoxy, 1-methyl-[1,2,4]triazol-5-ylmethylamino, 2-methyl-[1,2,4]triazol-5-ylmethylamino, 3,5-dimethylisoxazol-4-ylmethoxy, 5-methylisoxazol-3-ylmethoxy, morpholin-4-ylethoxy, OCH₂CN, 2-methylthiazol-4-ylmethoxy, 1-ethyl-[1,2,4]triazol-5-ylmethoxy, 1-isopropyl-[1,2,4]triazol-5-ylmethoxy, 1-methyl-5-chloro-3-trifluoromethylpyrazol-4-ylmethoxy,
1,5-dimethylpyrazol-3-ylmethoxy, 1,3,dimethylpyrazol-5-ylmethoxy, 1-methyl-5-chloropyrazol-4-ylmethoxy, ethoxy, 2,2,2-trifluoroethoxy, 1-methylimidazol-2-ylmethoxy, 1-methyltetrazol-5-ylmethoxy, pyrazin-2-ylmethoxy, pyrid-4-ylmethoxy, pyrimidin-6-ylmethoxy, 2-chlorothien-5-ylmethoxy, isopropoxy, 1-benzylimidazol-2-ylmethoxy, fur-2ylmethoxy, fur-3-ylmethoxy, thien-3-ylmethoxy, isoxazol-3-ylmethoxy, isoxazol-5-ylmethoxy,
isoxazol-4-ylmethoxy, pyrimidin-5-ylmethoxy, oxazol-5-ylmethoxy, 1-dimethylaminoethyl-[1,2,4]triazin-5-ylmethoxy, 1-methylimidazol-5-ylmethoxy, 1-methylpyrazol-3-ylmethoxy, 1-methylpyrazol-5-ylmethoxy, 3-methylisothiazol-5-ylmethoxy, thien-2-ylmethoxy, 1-methyl-[1,2,3]triazol;-4-ylmethylamino, thiazol-4-ylmethoxy, N-methyl-N-1-methyl-[1,2,3]triazol-4-ylmethylamino, isoxazol-5-ylmethylamino, pyrid-3-ylethoxy, pyrid-4-ylethoxy, 4-methylthiazol-5-ylethoxy, OCH₂CF₂H, OCH₂CH₂OCH₃, OCH₂CH₂N(CH₃)₂, 3-trifluoromethylpyrid-6-ylmethoxy, 2-trifluoromethylpyrid-5-ylmethoxy,
4-methylthiazol-5-ylmethoxy, 1-methylpyrazol-4-ylmethoxy, oxazol-5-ylmethylamino, CH₂OH, pyrid-3-ylmethylamino, benzimidazol-2-ylmethoxy, 4-trifluoromethylpyrid-6-ylmethoxy and 3H-imidazo[4,5-b]pyridin-2-ylmethoxy.

p is preferably 0 or 1. p may be 0. p may be 1.

A subclass of the compounds of formula I are defined by formula IIA: 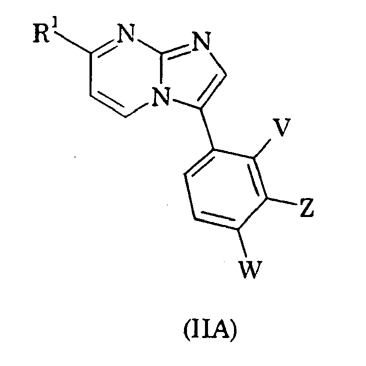 wherein R¹, V, W and Z have the same meanings as before.

In a particular embodiment of this subclass, R¹ is trifluoromethyl, V is H, Cl or F, and W is H, Cl, F or methyl, and at least one of V and W is H.

Within this subclass, preferred examples of groups represented by Z include H, Br, Cl, F, CN, NO₂, CF₃, OCF₃, CF₂H, SCF₃, amino, t-butylamino, dimethylamino, morpholin-4-yl, methyl, methoxymethyl, isopropyl, methoxy, methylthio, acetamido, N,N-dimethylcarbamoyl, methanesulphonyl, N,N-dimethylaminosulphonyl, 3-pyridoyl, (2-methyl-[1,2,4]triazol-3-yl)methoxy and (1-methyl-[1,2,4]triazol-3-yl)methoxy.

Z may have any of the values in the individual imidazopyrimidines disclosed herein. Likewise for R¹.

A second subclass of the compounds of formula I is defined by formula IIB: 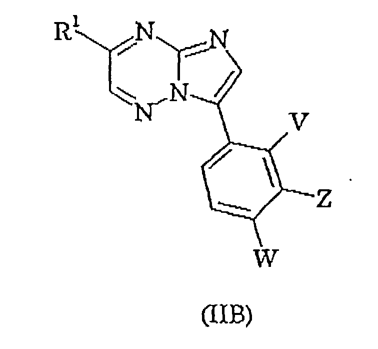 wherein R¹, V, W and Z have the same meanings as before.

In a particular embodiment of this subclass, R¹ is trifluoromethyl and one of V and W is H while the other is H, Cl or F.

Within this subclass, preferred examples of groups represented by Z include heteroarylmethoxy groups, such as (1,5-dimethylpyrazol-4-yl)methoxy, and (heteroarylmethyl)amino groups, such as (3-pyridylmethyl)amino, (1,5-dimethylpyrazol-4-yliuethyl)amino and (1-methyl-5-trifluoromethylpyrazol-4-ylmethyl)amino.

Z may have any of the values in the individual imidazotriazines disclosed herein. Likewise for R¹.

Examples of specific compounds in accordance with the invention include:
3-(3-trifluoromethylphenyl)-7-trifluoromethylimidazo[1,2-*a*]pyrimidine;
3-[3-cyanophenyl]-7-trifluoromethylimidazo[1,2-*a*]pyrimidine;
7-{3-[(1,5-dimethyl-1*H*-pyrazol-4-yl)methoxy]-4-fluorophenyl}-3-trifluoromethylimidazo[1,2-*b*][1,2,4]triazine;
N-[2-fluoro-5-(3-trifluoromethylimidazo[1,2-*b*][1,2,4]triazin-7-yl)phenyl]-N-(3-pyridinylmethyl)amine;
N-(1,5-dimethyl-1*H*-pyrazol-4-ylmethyl)-N-[2-fluoro-5-(3-trifluoromethylimidazo[1,2-*b*][1,2,4]triazin-7-yl)phenyl]amine;
7-[4-fluoro-3-(1-methyl-5-trifluoromethyl-1*H*-pyrazol-4-ylmethoxy)phenyl]-3-trifluoromethylimidazo[1,2-*b*][1,2,4]triazine;
3-[3-(1-methyl-1*H*-[1,2,4]triazol-3-ylmethoxy)phenyl]-7-trifluoromethylimidazo[1,2-*a*]pyrimidine;
3-(2-fluoro-3-trifluoromethylphenyl)-7-trifluoromethylimidazo[1,2-*a*]pyrimidine;
3-(3-trifluoromethoxyphenyl)-7-trifluoromethylimidazo[1,2-*a*]pyrimidine; and pharmaceutically acceptable salts thereof
Further examples include:
7-(4-fluoro-3-methoxyphenyl-3-trifluoromethylimidazo[1,2-*b*][1,2,4]triazine;
N-[2-fluoro-5-(3-trifluoromethylimidazo[1,2,*b*][1,2,4]triazin-7-yl)phenyl]*-N* methyl-N-(3-pyridinylmethyl)amine;
7-[4-fluoro-3-(pyridin-2-ylethoxy)phenyl]-3-(trifluoromethyl)imidazo[1,2-*b*][1,2,4]triazine;
2-fluoro-5-[3-(trifluoromethyl)imidazo[1,2-*b*][1,2,4]triazin-7-yl]benzaldehyde;
3-(3-trifluoromethylphenyl)imidazo[1,2-*a*]pyrimidine;
7-[3(1*H*-benzimidazol-2-ylmethoxy)-4-fluorophenyl]-3-trifluoromethylimidazo[1,2-*b*] [1,2,4]triazine;
7-(3-chlorophenyl)-3-trifluoromethylimidazo[1,2-*b*][1,2,4]triazine;
3-trifluoromethyl-7-(3-trifluoromethylsulfanylphenyl)imidazo[1,2-*b*][1,2,4]triazine;
7-[3-(1-methyl-1H-[1,2,4]triazol-3-ylmethoxy)phenyl]-3-trifluoromethylimidazo[1,2-*b*] [1,2,4]triazine;
and pharmaceutically acceptable salts thereof.

The compounds in accordance with the present invention have a good binding affinity (Ki) for the α5 subunit of the GABA_{A} receptor. In a preferred embodiment the compounds in accordance with the invention are binding selective for the α5 subunit relative to the α1, α2 and α3 subunits. In another preferred embodiment the compounds are functionally selective for the α5 subunit as partial or full inverse agonists whilst substantially being antagonists at the α1, α2 and α3 subunits.

The invention provides pharmaceutical compositions comprising one or more compounds of this invention and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration-by-inhalation or insufflation. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums or surfactants such as sorbitan monooleate, polyethylene glycol, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

For the enhancement of cognition, a suitable dosage level is about 0.01 to 250 mg/kg per day, preferably about 0.01 to 100 mg/kg of body weight per day, especially about 0.01 to 5 mg/kg of body weight per day, more particularly from 0.02 to 2.5 mg/kg of body weight per day. The compounds may be administered on a regimen of 1 to 4 times per day. In some cases, however, dosage outside these limits may be used. A typical body weight is 70 kg.

The compounds in accordance with the present invention may be prepared by a process which comprises reacting a compound of formula III with a compound of formula IV: 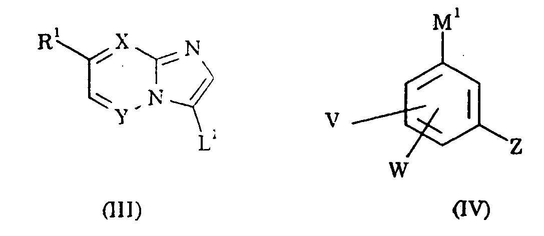 wherein V, W, X, Y, Z and R¹ are as defined above, L¹ represents a suitable leaving group, and M¹ represents a boronic acid moiety -B(OH)₂ or a cyclic ester thereof formed with an organic diol, e.g. pinacol or neopentyl glycol, or M¹ represents -Sn(Alk)₃ in which Alk represents a C₁₋₆ alkyl group, typically n-butyl; in the presence of a transition metal catalyst.

The leaving group L¹ is typically a halogen atom, e.g. bromo.

The transition metal catalyst of use in the reaction between compounds III and IV is suitably tetrakis(triphenylphosphine)-palladium(0). The reaction is conveniently carried out at an elevated temperature in a solvent such as *N,N*-dimethylacetamide 1,2-dimethoxyethane, tetrahydrofuran or 1,4-dioxane, advantageously in the presence of potassium phosphate or sodium carbonate (when M¹ is -B(OH)₂ or ester thereof), or copper(1) iodide (when M¹ is Sn(Alk)₃). Alternatively, the transition metal catalyst employed may be [1,1'-bis(diphenylphosphino)ferrocene]dichloropalladium(II) (Pd(dppf)Cl₂), in which case the reaction may conveniently be carried out at an elevated temperature in a solvent such as *N,N*-dimethylformamide, typically in the presence of potassium phosphate.

Where M¹ in the intermediates of formula IV above represents a boronic acid moiety -B(OH)₂ or a cyclic ester thereof formed with pinacol or neopentyl glycol, the relevant compound IV may be prepared by reacting bis(pinacolato)diboron or bis(neopentyl glycolato)diborane with a compound of formula IVA: 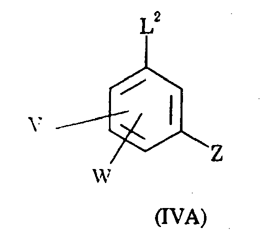 wherein V, W and Z are as defined above, and L² represents a suitable leaving group; in the presence of a transition metal catalyst.

Typical leaving groups represented by L² include trifluoromethanesulfonyloxy (triflyloxy); or a halogen atom such as bromo.

The transition metal catalyst of use in the reaction between bis(pinacolato)diboron or bis(neopentylglycolato)diborane and compound IVA is suitably [1,1'-bis(diphenylphosphino)ferrocene]dichloro-palladium(II). The reaction is conveniently carried out at an elevated temperature in a solvent such as 1,4-dioxane, optionally in admixture with dimethylsulfoxide, typically in the presence of Pd(dppf)Cl₂ and/or potassium acetate.

Where L² in the intermediates of formula IVA above represents triflyloxy, the relevant compound IVA may be prepared by reacting the corresponding compound of formula IV in which L² represents OH with *N-*phenyl-triflylimide, typically in the presence of triethylamine; or with triflic anhydride, typically in the presence of pyridine

Where L¹ in the intermediates of formula III above represents bromo, this compound may be prepared by bromination of the corresponding compound of formula V: 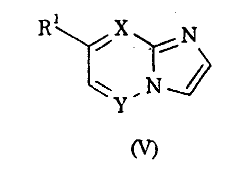 wherein X, Y and R¹ are as defined above; typically by treatment with bromine in acetic acid, in the presence of sodium acetate and optionally also potassium bromide.

The intermediates of formula V may be prepared by reacting chloroacetaldehyde or bromoacetaldehyde, or an acetal derivative thereof, e.g. the dimethyl or diethyl acetal thereof, with the requisite compound of formula VI: 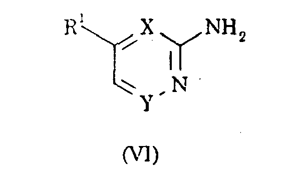 wherein X, Y and R¹ are as defined above.

Where chloroacetaldehyde or bromoacetaldehyde is utilised as one of the reactants, the reaction is conveniently carried out by heating the reactants under basic conditions in a suitable solvent, e.g. sodium methoxide or sodium hydrogencarbonate in a lower alkanol such as methanol and/or ethanol at the reflux temperature of the solvent. Where an acetal derivative of chloroacetaldehyde or bromoacetaldehyde, e.g. the dimethyl or diethyl acetal thereof, is utilised as one of the reactants, the reaction is conveniently effected by heating the reactants under acidic conditions in a suitable solvent, e.g. aqueous hydrobromic acid in a lower alkanol such as methanol or ethanol, typically at the reflux temperature of the solvent.

In a still further procedure, the compounds according to the present invention may be prepared by a process which comprises reacting a compound of formula VI as defined above with a compound of formula VII: 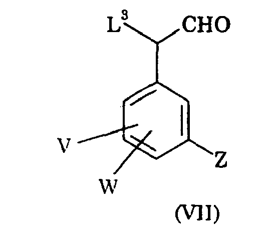 wherein V, W and Z are as defined above, and L³ represents a suitable leaving group; under conditions analogous to those described above for the reaction between bromoacetaldehyde and compound VI.

The leaving group L³ is suitably a halogen atom, e.g. bromo.

In a yet further procedure, the compounds according to the present invention wherein R¹ represents an aryl or heteroaryl moiety may be prepared by a process which comprises reacting a compound of formula VIII with a compound of formula IX: 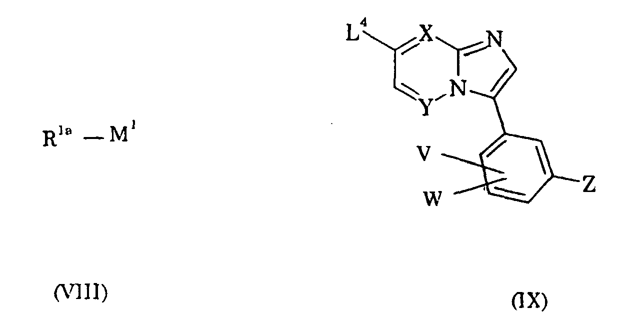 wherein V, W, X, Y, Z and M¹ are as defined above, R^{1a} represents an aryl or heteroaryl moiety, and L⁴ represents a suitable leaving group; in the presence of a transition metal catalyst.

The leaving group L⁴ is typically a halogen atom, e.g. chloro.

The transition metal catalyst of use in the reaction between compounds VIII and IX is suitably tetrakis(triphenylphosphine)-palladium(0), in which case the reaction is conveniently effected at an elevated temperature in a solvent such as *N,N*-dimethylacetamide, typically in the presence of potassium phosphate or in the presence of lithium chloride and copper(I) iodide. Alternatively, the transition metal catalyst may suitably be tris(dibenzylideneacetone)palladium(0), in which case the reaction is conveniently effected at an elevated temperature in a solvent such as 1,4-dioxane, typically in the presence of tri-*tert-*butylphosphine and cesium carbonate.

Where L⁴ in the compounds of formula IX above represents a halogen atom, these compounds correspond to compounds of formula I as defined above wherein R¹ represents halogen, and they may therefore be prepared by any of the methods described above for the preparation of the compounds according to the invention.

In an alternative process a compound in accordance with the invention can be made by reacting a compound of formula V with a compound of formula X: 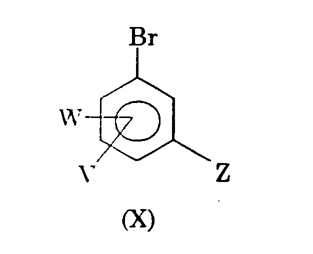 in which W, V and Z are as defined above, in the presence of palladium acetate, triphenylphosphine and cesium carbonate in an anhydrous solvent such as dry dioxan at about 90°C for about 2 h.

Where they are not commercially available, the starting materials of formula IV, IVA, VI, VII, VIII and X may be prepared by methods analogous to those described in the accompanying Examples, or by standard methods well known from the art.

It will be understood that any compound of formula I initially obtained from any of the above processes may, where appropriate, subsequently be elaborated into a further compound of formula I by techniques known from the art. For example, a compound of formula I wherein R¹ represents C₁₋₆alkoxycarbonyl initially obtained may be hydrolysed to the corresponding carboxylic acid, or reacted with ammonia to provide the corresponding carboxamide, or reduced with lithium aluminium hydride to the corresponding compound of formula I wherein R¹ represents hydroxymethyl. The latter compound may then be oxidised to the corresponding compound of formula I wherein R¹ represents formyl by treatment with manganese dioxide. The formyl derivative thereby obtained may be condensed with a hydroxylamine derivative of formula H₂N-OR^{b} to provide a compound of formula I wherein R¹ represents -CH=NOR^{b}. Furthermore, a compound of formula I wherein R¹ represents -CH=NOH may be treated with triethylamine in the presence of 1,1'-carbonyldiimidazole to afford a corresponding compound of formula I wherein R¹ represents cyano. Alternatively, the compound of formula I wherein R¹ represents formyl may be reacted with a Grignard reagent of formula RₐMgBr to afford a compound of formula I wherein R¹ represents -CH(OH)R^{a}, and this compound may in turn be oxidised using manganese dioxide to the corresponding compound of formula I wherein R¹ represents -COR^{a}. The latter compound may then be condensed with a hydroxylamine derivative of formula H₂N-OR^{b} to provide a compound of formula I wherein R¹ represents -CR^{a}=NOR^{b}.

Similarly, compounds of formula I in which Z represents OCH₃ may be converted to the corresponding phenols (1, Z=OH) by treatment with HBr, typically in acetic acid at reflux, or by treatment with boron tribromide, typically in dichloromethane at -78°C. The phenols may be reacted with HO(CH₂)ₙheteroaryl in the presence of a dialkylazodicarboxylate (e.g. diisopropylazodicarboxylate) and triphenylphosphine to provide the compounds of formula I in which Z represents -O(CH₂)ₙheteroayl, where n has the same meaning as before. The reaction is typically-carried out at ambient temperature in THF. Alternatively, the same products may be obtained by reaction-of the phenols (I, Z=OH) with L⁵-(CH₂)ₙheteroaryl in the presence of base, where L⁵ is a leaving group such as halogen, tosylate, mesylate or triflate, and n has the same meaning as before.

Similarly, the compounds of formula I in which Z represents NO₂ may be reduced to the corresponding anilines (I, Z=NH₂) by standard methods (e.g. using tin(II)chloride), and the amino group converted to a variety of derivatives by alkylation, acylation or diazotisation in using standard methods. In particular, the anilines may be reacted with the aldehydes heteroaryl(CH₂)ₙ₋₁CHO in the presence of sodium triacetoxyborohydride to provide the compounds of formula I wherein Z represents -NH(CH₂)ₙheteroaryl, where n has the same meaning as before. This process may be repeated to provide compounds wherein Z represents -NR'(CH₂)ₙheteroaryl wherein R is as defined above.

Where the above-described processes for the preparation of the compounds according to the invention give rise to mixtures of stereoisomers, these isomers may be separated by conventional techniques such as preparative chromatography. The novel compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as (-)-di-*p*-toluoyl-d-tartaric acid and/or (+)-di-*p*-toluoyl-1-tartaric acid, followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in *Protective Groups in Organic Chemistry,* ed. J.F.W. McOmie, Plenum Press, 1973; and T.W. Greene & P.G.M. Wuts, *Protective Groups in Organic Synthesis,* John Wiley & Sons, 1991. The protecting groups may be removed at a convenient subsequent stage using methods known from the art.

The following Examples illustrate the preparation of compounds according to the invention.

The compounds in accordance with this invention potently inhibit the binding of [3H]-flumazenil to the benzodiazepine binding site of human GABA_{A} receptors containing the α5 subunit stably expressed in Ltk- cells.

### Reagents

- Phosphate buffered saline (PBS).
- Assay buffer: 10 mM KH₂PO₄, 100 mM KCl, pH 7.4 at room temperature.
- [3H]-Flumazenil (18 nM for α1β3γ2 cells; 18 nM for α2β3γ2 cells; 10 nM for α3β3γ2 cells; 10 nM for α5β3γ2 cells) in assay buffer.
- Flunitrazepam 100 µM in assay buffer.
- Cells resuspended in assay buffer (1 tray to 10 ml).

### Harvesting Cells

Supernatant is removed from cells. PBS (approximately 20 ml) is added. The cells are scraped and placed in a 50 ml centrifuge tube. The procedure is repeated with a further 10 ml of PBS to ensure that most of the cells are removed. The cells are pelleted by centrifuging for 20 min at 3000 rpm in a benchtop centrifuge, and then frozen if desired. The pellets are resuspended in 10 ml of buffer per tray (25 cm x 25 cm) of cells.

### Assay

Can be carried out in deep 96-well plates or in tubes. Each tube contains:
- 300 µl of assay buffer.
- 50 µl of [3H]-flumazenil (final concentration for α1β3γ2: 1.8 nM; for α2β3γ2: 1.8 nM; for α3β3γ2: 1.0 nM; for α5β3γ2: 1.0 nM).
- 50 µl of buffer or solvent carrier (e.g. 10% DMSO) if compounds are dissolved in 10% DMSO (total); test compound or flunitrazepam (to determine non-specific binding), 10 µM final concentration.
- 100 µl of cells.

Assays are incubated for 1 hour at 40°C, then filtered using either a Tomtec or Brandel cell harvester onto GF/B filters followed by 3 x 3 ml washes with ice cold assay buffer. Filters are dried and counted by liquid scintillation counting. Expected values for total binding are 3000-4000 dpm for total counts and less than 200 dpm for non-specific binding if using liquid scintillation counting, or 1500-2000 dpm for total counts and less than 200 dpm for non-specific binding if counting with meltilex solid scintillant. Binding parameters are determined by non-linear least squares regression analysis, from which the inhibition constant Ki can be calculated for each test compound.

The compounds of the accompanying Examples were tested in the above assay, and all were found to possess a Ki value for displacement of [3H]Ro 15-1788 from the α5 subunit of the human GABA_{A} receptor of 100 nM or less, and some were at 1 nM or less.

The compounds of the present invention can be shown to enhance cognition in the rat water maze test (Morris, Learning and Motivation, 1981, 12, 239ff). This has been demonstrated for at least one compound described herein. Further details of methodology for demonstrating that the present compounds enhance cognition can be found in WO-A-9625948.

### Example 1

### 3-(3-Trifluoromethylphenyl)-7-trifluoromethylimidazo[1,2-a]pyrimidine

### Step 1: 7-Trifluoromethilimidazo[1,2-a]pyrimidine

A mixture of 2-amino-4-trifluoromethylpyrimidine (prepared according to Zanatta *et al.* in *J. Heterocyclic Chem.,* 1997,34(2), 509-513) (500 mg, 3.1 mmol) and bromoacetaldehyde diethyl acetal (1.38 mL, 9.2 mmol) in ethanol (10 mL) was treated with hydrobromic acid (0.5 mL of a 48% aqueous solution) and then heated at 70°C for 12 h. The reaction was cooled to ambient temperature then pre-adsorbed onto silica. Purification by chromatography on silica eluting with dichloromethane (containing 1% conc. ammonia) on a gradient of methanol (1-5%) afforded 7-trifluoromethylimidazo[1,2-*a*]pyrimidine (500 mg, 87%) as a cream-coloured solid: δ (400 MHz, CDCl₃) 7.22 (1H, d, *J* 7), 7.74 (1H, d, *J* 1), 8.03 (1H, d, *J* 1), 8.67 (1H, d, *J* 7).

### Step 2: 3-Bromo-7-trifluoromethylimidazo[1,2-a]pyrimidine

7-Trifluoromethylimidazo[1,2-*a*]pyrimidine (2.0 g, 10.7 mmol) and sodium acetate (1.1 g, 13.4 mmol) were dissolved in methanol (30 mL) which had been saturated with potassium bromide and this mixture was cooled to - 10°C before dropwise addition of bromine (1.86 g, 11.7 mmol) over 5 min. On complete addition, the mixture was quenched by addition of 1M sodium sulfite solution (2 mL) and the solvent removed *in vacuo.* The residue was treated with water (100 mL) and saturated sodium hydrogencarbonate solution (100 mL) and extracted with ethyl acetate (3 x 100 mL). The organics were combined then washed with brine (100 ml), dried over anhydrous sodium sulfate and evaporated to give an off-white solid. This solid was purified by silica gel chromatography eluting with dichloromethane and 1% conc. ammonia on a gradient of methanol (1-2%) to give 3-bromo-7-trifluoromethylimidazo[1,2-*a*]pyrimidine (1.98 g) as a white solid: δ (400 MHz, CDCl₃) 7.35 (1H, d, *J* 7), 8.02 (1H, s), 8.62 (1H, d, *J* 7).

### Step 3: 3-(3-Trifluoromethylphenyl)-7-trifluoromethylimidazo[1,2-a]pyrimidine

A mixture of 3-bromo-7-trifluoromethylimidazo[1,2-*a*]pyrimidine (300 mg, 1.12 mmol), 3-trifluoromethylbenzeneboronic acid (429 mg, 2.24 mmol), 2N Na₂CO₃ solution (2.24 mL) and THF (4.5 mL) were degassed with a stream of N₂ for 5 min and then *tetrakis*(triphenylphosphine)palladium(0) (130 mg, 10 mol %) was added and the reaction was heated at 70°C for 90 min. EtOAc (70 mL) was added and the mixture separated, washed with brine (20 mL), dried (MgSO₄) and concentrated under reduced pressure while dry loading onto MgSO₄. The residue was purified by column chromatography on silica using 40% Et₂O in *iso*-hexanes. The resulting material was further purified by column chromatography on silica using 35% EtOAc in *iso*-hexanes to give the desired *imidazopyrimidine* (83 mg, 22 %). ¹H NMR (400 MHz, d₆-DMSO) δ 7.54 (1H, d, *J* = 7.1 Hz), 7.76-7.90 (2H, m), 8.04-8.15 (2H, m), 8.38 (1H, s), 9.30 (1H, d, *J* = 7.1 Hz). m/z (ES⁺) 332 (M+H⁺).

### Example 2

### 3-[3-Cyanophenyl]-7-trifluoromethylimidazo[1,2-a]pyrimidine

### Step 1: 3-(5,5-Dimethyl-1,3,2-dioxaborinan-2-yl)benzonitrile

A mixture of 3-iodobenzonitrile (2.0 g, 8.7 mmol), bis-(neopentylglycolato)diborane (2.17 g, 9.6 mmol), KOAc (2.6 g, 26.2 mmol) and Pd(dppf)Cl₂ (356 mg, 5 mol%) in 1,4-dioxane (60 mL) was degassed with a stream of N₂ for 30 min and then heated at 110°C for 16 h. The reaction mixture was concentrated under reduced pressure and diethyl ether (150 mL) was added. This mixture was extracted with 4N NaOH (3 x 50 mL) and the combined basic extracts were neutralised with conc. HCl and then extracted with DCM (3 x 100 mL). The combined organic filtrates were washed with brine (50 mL) and dried (MgSO₄) to yield the desired *boronate ester* (1.82 mg, 97 %). ¹H NMR (360 MHz, CDCl₃) δ 1.03 (6H,s),3.78(4H,s);7.44(1H,t,*J*=7.6Hz),7.67(1H,t,*J*=7.6 Hz), 7.99 (1H, d, *J* = 7.6 Hz), 8.08 (1H, s).

### Step 2: 3-[3-Cyanophenyl]-7-trifluoromethylimidazo[1,2-a]pyrimidine

The reaction was carried out as described in step 3 of Example 1 using 3-bromo-7-trifluoromethylimidazo[1,2-*a*]pyrimidine (300 mg, 1.1 mmol), the boronate ester (487 mg, 2.2 mmol), 2N Na₂CO₃ solution (2.2 mL) and THF (4.5 mL) *tetrakis*(triphenylphosphine) palladium(0) (130 mg, 10 mol%) at 70°C for 90 min. After work up the residue was purified twice by column chromatography on silica using 40% Et₂O in toluene and then 55% EtOAc in *iso*-hexanes to give the desired *imidazopyrimidine* (40 mg, 12 %). ¹H NMR (400 MHz, d₆-DMSO) δ 7.55 (1H, d, *J* = 7.0 Hz), 7.79 (1H, t, *J* = 7.8 Hz), 7.95 (1H, d, *J* = 7.8 Hz), 8.10 (1H, d, *J* = 7.8 Hz), 8.29 (1H, s), 8.38 (1H, s), 9.41 (1 H, d, *J* = 7.0 Hz). *m*/*z* (ES⁺) 289 (M+H⁺).

### Example 3

### 7-(4-Fluoro-3-methoxyphenyl)-3-trifluoromethylimidazo[1,2-b] [1,2,4]triazine

### Step 1: 3-Amino-5-trifluoromethyl-1,2,4-triazine

To a stirred solution of sodium acetate trihydrate (22.62 g, 166.2 mmol) in water (80 mL) was added 1,1-dibromo-3,3,3-trifluoroacetone (21.57g, 79.9mmol). The solution was heated at reflux under nitrogen for 30 min, then allowed to cool to room temperature before adding solid aminoguanidine bicarbonate (10.88 g, 79.9 mmol). The resulting pale yellow solution (pH 5) was stirred at room temperature for 3 h, then 4 N aqueous NaOH solution (40 mL, 160 mmol) was added causing a precipitate to appear. The mixture (pH 10) was stirred under nitrogen for a further 39h. The solid was collected by filtration, washed with water and dried at 60°C under vacuum-to-give 6.96g of a mixture of two isomers in a 28:72 ratio. This was further purified by flash chromatography (silica gel, 30% EtOAc/isohexane), then recrystallised from ethanol to afford 3.53 g (27%) of the title compound: ¹H NMR (400 MHz, DMSO-*d₆*) δ 8.00 (2H, br s), 9.08 (1H, s).

### Step 2: 3-Trifluoromethylimidazol[1,2-b][1,2,3]triazine

A stirred mixture of bromoacetaldehyde diethyl acetal (2.30 mL, 14.8 mmol) in concentrated hydrobromic acid (0.73 mL) and water (0.73 mL) was heated at reflux for 2 h, then poured into ethanol (25 mL). The solution was neutralised to pH 7 with solid sodium hydrogencarbonate, then filtered. To the filtrate was added 3-amino-5-trifluoromethyl-1,2,4-triazine (1.08 g, 6.14 mmol) and the mixture was stirred at 60°C for 20 h, then 80°C for 23 h. The mixture was evaporated *in vacuo,* and the residue was purified by flash chromatography (silica gel, 35-50% EtOAc/isohexane) to give 0.259 g (22%) of the title compound: ¹H NMR (360 MHz, CDCl₃) δ 8.20 (1H, d, *J* 0.8 Hz), 8.30 (1H, d, *J* 0.9 Hz), 8.73 (1H, s).

### Step 3: 7-(4-Fluoro-3-methoxyphenyl)-3-trifluoromethylimidazo[1,2-b][1,2,4]triazine

A mixture of 3-trifluoromethylimidazo[1,2-*b*][1,2,4]triazine (3.2g, 17 mmol), palladium acetate (185 mg, 5 mol%), triphenylphosphine (672 mg, 15mol%), cesium carbonate (8.2 g, 25 mmol) and 4-bromo-1-fluoro-2-methoxybenzene (4.1 g, 20 mmol) in dry 1,4-dioxan (100 mL) under an inert atmosphere was heated at 90°C for 20 h. The cooled reaction mixture was diluted with ethyl acetate, filtered through Hyflo and solvents removed *in vacuo.* The residue was redissolved in ethyl acetate, washed with water and saturated brine then dried (MgSO₄) filtered and concentrated in *vacuo.* Crystallisation from ethyl acetate, washing with ethanol and drying gave 7-(4-fluoro-3-methoxyphenyl)-3-trifluoromethylimidazo[1,2-*b*][1,2,4]triazine as a coloured solid (3.5 g). ¹H NMR (400 MHz, CDCl₃) δ 4.01 (3H, s), 7.26 (1H, dd, *J* 11.0 and 8.6), 7.61-7.64 (1H, m), 7.76 (1H, dd, *J* 2.2 and 8.0), 8.57 (1H, s), 8.81 (1H, s).

### Example 4

### 7-{[3-(1,5-Dimethyl-1H-pyrazol-4-yl)methoxy]-4-fluorophenyl}-3-trifluoromethylimidazo[1,2-b]1,2,4]triazine

### Step 1: Intermediate 1: (1,5-Dimethyl-1H-pyrazol-4-yl)-methanol

Sodium borohydride (0.75 g, 19.3 mmol) was added in portions over 15 min to a stirred solution of 1,5-dimethyl-1*H*-pyrazole-4-carbaldehyde (2.40 g, 19.3 mmol; *Zh. Org. Khim.* (1973), 9(4), 815-817) in dry methanol (30 mL) at 0°C under an inert atmosphere. After 1h the solution was allowed to warm to ambient temperature then partitioned between diethyl ether and water. Aqueous washings were concentrated *in vacuo* and extracted with dichloromethane. These extracts were washed with a minimum of saturated brine then dried (MgSO₄), filtered and evaporated *in vacuo* to give (1,5-dimethyl-1*H*-pyrazol-4-yl)-methanol as a solid (1.2 g). ¹H NMR (360 MHz, CDCl₃) δ 1.30 (1H, s), 2.28 (3H, s), 3.78 (3H, s), 4.51 (2H, s), 7.41 (1H, s). *m*/*z* (ES⁺) 127 (M+H⁺).

### Step 2: 2-Fluoro-5-(3-trifluoromethylimidazo[1,2-b][1,2,4]triazin-7-yl)-phenol

Boron tribromide (4.5 mL, 47 mmol) was added dropwise to a stirred solution of 7-(4-fluoro-3-methoxyphenyl)-3-trifluoromethylimidazo[1,2-*b*][1,2,4]triazine (2.5 g, 8 mmol) in dry dichloromethane (100 mL) at -78°C. The cooling bath was removed after 30 min and the mixture allowed to warm to ambient temperature. Reaction was quenched using methanol at 0°C then poured into a saturated aqueous solution of sodium hydrogen carbonate. The precipitate was filtered off, washed with water and dried *in vacuo* at 80°C to give 2-fluoro-5-(3-trifluoromethylimidazo[1,2-*b*][1,2,4]triazin-7-yl)-phenol *(Intermediate 1)* as an orange solid (1.8 g). ¹H NMR (400 MHz, d6-DMSO) δ 7.38 (1H, dd, *J* 8.6 and 11.0), 7.60-7.63 (1H, m), 7.88 (1H, dd, *J* 2.2 and 8.4), 8.83 (1H, s), 9.32 (1H, s), 10.27 (1H, s). *m*/*z* (ES⁺) 299 (M+H⁺).

### Step 3: 7-{[3-(1,5-Dimethyl-1H-pyrazol-4-yl)methoxy]-4-fluorophenyl}-3-trifluoromethylimidazo[1,2-b][1,2,4]triazine

Diisopropylazodicarboxylate (0.85 mL, 3.99 mmol) was added dropwise over 10 min to a stirred solution of 2-fluoro-5-(3-trifluoromethylimidazo[1,2-*b*][1,2,4]triazin-7-yl)-phenol (1.0 g, 3.35 mmol), triphenylphosphine (1.05 g, 3.97 mmol) and (1,5-dimethyl-1H-pyrazol-4-yl)-methanol (0.50 g, 3.97 mmol) in dry tetrahydrofuran (50 mL). After stirring at ambient temperature overnight the mixture was adsorbed onto silica and solvent removed *in vacuo.* Purification by column chromatography on a gradient of ethyl acetate-isohexane (20-100%) and trituration with 50% diethyl ether-isohexane gave 7-{[3-(1,5-dimethyl-1*H-*pyrazol-4-yl)methoxy]-4-fluorophenyl}-3-trifluoromethylimidazo[1,2-*b*][1,2,4]triazine as an orange solid (0.68 g). ¹H NMR (400 MHz, d6-DMSO) δ 2.28 (3H, s), 3.73 (3H, s), 5.11 (2H, s), 7.45 (1H, dd, *J* 8.6 and 11.3), 7.48 (1H, s), 7.85-7.88 (1H, m), 8.11 (1H, dd, *J* 2.2 and 8.0), 8.97 (1H, s), 9.36 (1H, s). *m*/*z* (ES⁺) 407 (M+H⁺).

### Preparative Example 5

### Methyl 3-(3-trifluoromethylphenyl)imidazo[1,2-a]pyridine-7-carboxylate

A mixture of methyl 3-bromoimidazo[1,2-*a*]pyridine-7-carboxylate (WO0138326) (250 mg, 0.98 mmol), 3-trifluoromethylbenzeneboronic acid (280 mg, 1.47 mmol), 2M Na₂CO₃ solution (1.5 mL) and THF (5 mL) was degassed with a stream of N₂ for 5 min.

*Tetrakis*(triphenylphosphine)palladium(0) (57 mg, 10 mol %) was added and the reaction was heated at reflux for 3 h. The mixture was then partitioned between CH₂Cl₂ (20 mL) and water (20 mL) and the organics were washed with brine (20 mL), dried (MgSO₄) and concentrated under reduced pressure. The residue was purified by column chromatography on silica using 80% EtOAc in *iso*-hexanes to afford the desired *imidazopyridine* (195 mg, 62 %). ¹H NMR (360 MHz, d₆-DMSO) δ 3.31 (3H, s), 7.24 (1H, dd, *J* 2.0, 7.2 Hz), 7.80-7.87 (2H, m), 8-05-8.08 (2H, m), 8.13 (1H, s), 8.19-8.21 (1H, m) 8.75 (1H, d, *J* 7.2 Hz). m/z (ES⁺) 321 (MH⁺).

### [Example 6 deleted]

### Example 7

### N-[2-Fluoro-5-(3-trifluoromethylimidazo[1,2-b][1,2,4]triazin-7-yl)phenyl]-N-(3-pyridinylmethyl)amine

### Step 1: 2-Fluoro-5-(3-trifluoromethylimidazo[1,2-b][1,2,4]triazin-7-yl)phenylamine

A mixture of 4-bromo-1-fluoro-2-nitrobenzene (10 g, 0.045 mol), potassium acetate (13.3 g, 0.135 mol) and *bis*(neopentylglycolato)diboron (10.6 g, 0.047 mol) in 1,4-dioxane (100 mL) was degassed with N₂. [1,1'-Bis(diphenylphosphino)ferrocene]dichloropalladium (II), complex with DCM (1:1) (0.92 g, 1.1 mmol) was added and the mixture heated at reflux for 24 h. The mixture was evaporated *in vacuo* and the residue partitioned between DCM and water then filtered to remove an undissolved black solid. The DCM layer was retained, dried (MgSO₄), filtered and evaporated to dryness to give 2-(4-fluoro-3-nitrophenyl)-5,5-dimethyl-[1,3,2]dioxaborinane as a brown oil (17 g) which was used without purification in the next step. ¹H NMR (360 MHz, CDCl₃) δ 1.03 (6H, s), 3.78 (4H, s), 7.21-7.26 (1H, m), 7.99-8.05 (1H, m), 7.44-8.47 (1H, m).

A mixture of 7-bromo-3-trifluoromethylimidazo[1,2-*b*][1,2,4]triazine (10 g, 0.037 mol), the foregoing boronate ester (13.5 g, 0.061 mol) and 2N Na₂CO₃ (61 mL, 0.12 mol) in THF (300 mL) was degassed with N₂. *Tetrakis*(triphenylphosphine)palladium(0) (2.14 g, 1.85 mmol) was added and the mixture heated at reflux for 24 h. The mixture was evaporated *in vacuo* and the residue partitioned between DCM and water. The aqueous layer was re-extracted with DCM and the combined organics washed twice with 4N NaOH, then twice with brine and dried (MgSO₄). The solvent was evaporated *in vacuo* to give the crude residue which was purified on silica eluting with DCM. 7-(4-Fluoro-3-nitrophenyl)-3-trifluoromethylimidazo[1,2-*b*][1,2,4]triazine was isolated as an orange solid (7.2 g) after trituration with ether, filtration and drying. A second crop (0.8 g, combined yield 65 %) was isolated by trituration of the evaporated liquors. MS (ES⁺) 328 (M +1).

A mixture of the foregoing nitro compound (1.25 g, 3.8 mmol), tin(II) chloride (3.0 g, 15.8 mmol) in EtOH (100 mL) was stirred at 25°C for 60h. Aqueous NH₃ (33%) (50 mL) was added, stirred for 5 min, then evaporated to dryness. The residue was triturated with diethyl ether and filtered. This procedure was repeated 3 times, MgSO₄ added, and the mixture evaporated to dryness and loaded onto a silica column. The column was eluted with 10% EtOAc/isohexane then 20% EtOAc/isohexane. The title compound was isolated as a yellow solid (300 mg, 27 %). ¹H NMR (400 MHz, *d*₆-DMSO) δ 5.42 (2H, s), 7.20 (2H, dd, *J* 8.5 & 11.4 Hz), 7.31 (1H, ddd, J = 2.2, 4.4 & 8.4 Hz), 7.58 (1H, dd, J 2.2 & 8.7 Hz), 8.73 (1H, s), 9.28 (1H; s). MS (ES⁺) 298 (M + 1).

### Step 2: N-[2-Fluoro-5-(3-trifluoromethylimidazo[1,2-b][1,2,4]triazin-7-yl)phenyl]-N-(3-pyridinylmethyl)amine

A solution of the foregoing aniline (150 mg, 0.51 mmol) and 3-pyridinecarboxaldehyde (54 mg, 0.51 mmol) in AcOH (10 mL) was stirred at 25°C for 7 h. Sodium triacetoxyborohydride (130 mg, 0.61 mmol) was added and the reaction stirred for 18 h. The mixture was evaporated to dryness, the residue partitioned between DCM and water and then neutralised with saturated aqueous NaHCO₃. The DCM layer was separated and the aqueous phase re-extracted with DCM. The combined organics were dried (MgSO₄), and concentrated to give the crude residue which was purified by silica chromatography eluting 10% diethyl ether/DCM then 1% MeOH/DCM to give an orange solid (102 mg, 52 %). ¹H NMR (400 MHz, *d*₆-DMSO) δ 4.47 (2H, d, J 6.2 Hz), 6.62-6.70 (1H, m), 7.24 (1H, dd, J = 8.2 & 11.7 Hz), 7.34-7.43 (2H, m), 7.51 (1H, dd, J 2.0 & 8.6 Hz), 7.85 (1H, d, J 7.8 Hz), 8.45 (1H, br s), 8.70 (1H, br s), 8.79 (1H, s), 9.21 (1H, s). MS (ES⁺) 389 (M + 1).

### Example 8

### N-(1,5-Dimethethyl-1H-pyrazol-4-ylmethyl)-N-[2-fluoro-5-(3-trifluoromethylimidazo[1,2-b][1,2,4]triazin-7-yl)phenyl]amine

The title compound was obtained as a red solid (50 mg, 46 %) using the procedure described in Example 7, Step 2 using 1,5-dimethyl-1*H-*pyrazole-4-carbaldehyde instead of 3-pyridinecarboxaldehyde. ¹H NMR (400 MHz, *d*₆-DMSO) δ 2.27 (3H, s), 3.66 (3H, s), 4.18 (2H, d, J 5.7 Hz), 5.92-5.97 (1H, m), 7.22 (1H, dd, J 8.4 & 11.7 Hz), 7.39 (1H, s), 7.42 (1H, dd, J 2.1, 4.4 & 8.3 Hz), 7.58 (1H, dd, 2.1 & 8.5 Hz), 8.85 (1H, s), 9.29 (1H, s). MS (ES⁺) 406 (M + 1).

### Example 9

### 7-[4-Fluoro-3-(1-methyl-3-trifluoromethyl-1H-pyrazol-4-ylmethoxy)phenyl]-3-trifluoromethylimidazo[1,2-b]triazine

A solution of 1-methyl-3-trifluoromethyl-1*H*-pyrazole-4-carboxylic acid ethyl ester (2.52 g, 11.3 mmol) in THF (40 mL) was added to a stirred solution of 1.0 M LiAlH₄ in diethyl ether (45 mL, 45 mmol). Upon complete addition the reaction was heated at 50°C for 5 h. The cooled reaction mixture was quenched cautiously by addition of water (10 mL). 2N HCl (100 mL) and DCM (100 mL) were added and the mixture stirred for 30 min. The organic phase was separated and the aqueous re-extracted with DCM. The combined organics were washed with brine, dried (MgSO₄) and concentrated *in vacuo.* The crude residue was purified on silica, eluting 5% MeOH/DCM, to give (1-methyl-5-trifluoromethyl-1*H-*pyrazol-4-yl)methanol as a pale yellow oil (1.46 g, 72 %). ¹H NMR (360 MHz, CDCl₃) δ 3.93 (3H. s). 4.66 (2H, s), 7.46 (1H, s).

The foregoing alcohol was reacted with 2-fluoro-5-(3-trifluoromethylimidazo[1,2-*b*][1,2,4]triazin-7-yl)phenol by the procedure described in Example 4, Step 3 using diethyl azodicarboxylate instead of diisopropyl azodicarboxylate. The title compound was isolated as a orange solid (65 mg, 70 %). ¹H NMR (400 MHz, *d*₆-DMSO) δ 3.93 (3H, s), 5.22 (2H, s), 7.46 (1H, dd, J 8.6, & 11.2 Hz), 7.90 (1H, ddd, J 2.0, 4.2 & 8.4 Hz), 8.07 (1H, dd, J 2 & 8 Hz), 8.14 (1H, s), 8.97 (1H, s), 9.36 (1H, s). MS (ES⁺) 461 ( M + 1).

### Example 10

### 3-[3-(1-Methyl-1H-[1,2,4]triazol-3-ylmethoxy)phenyl]-7-trifluoromethylimidazo[1,2-a]pyrimidine

### Step 1: 3-(3-Bromophenoxymethyl)-1-methyl-1H-[1,3,4]triazole

A mixture of 3-bromophenol (2.0 g, 11.6 mmol), 3-chloromethyl-1-methyl-1*H*-[1,2,4]triazole hydrochloride (WO 9850385) (1.94 g, 11.6 mmol) and K₂CO₃ (4.79 g, 34.7 mmol) in DMF (20 mL) was stirred overnight at 25°C under N₂**.** Water (200 mL) was added and the organics were extracted with EtOAc (2 x 100 mL). The combined organic extracts were washed with water (2 x 100 mL), brine (50 mL) then dried (MgSO₄) and concentrated under reduced pressure. The crude residue was purified by column chromatography on silica using 100% EtOAc to yield the *ether* (1.31g, 42 %). ¹H NMR (360 MHz, CDCl₃) δ 8.04 (1H, s), 7.20 (1H, s), 7.18-7.06 (2H, m), 6.97 (1H, d, *J* = 7.9 Hz), 5.12 (2H, s), 3.93 (3H, s).

### Step 2:3-[3-(5,5-Dimethyl-[1,3,2]dioxaborinan-2-yl)phenoxymethyl]-1-methyl-1H-[1,2,4]triazole

The reaction was carried out according to Example 2, Step 1 using 3-(3-bromophenoxymethyl)-1-methyl-1*H*-[1,2,4]triazole (1.31 g, 4.9 mmol), *bis*-(neopentylglycolato)diborane (1.16 g, 5.2 mmol), KOAc (1.44 g, 14.7 mmol) and Pd(dppf)Cl₂ (200 mg, 5 mol%) in 1,4-dioxane (70 mL) to yield the desired *boronate ester* (1.22 g, 83 %). ¹H NMR (360 MHz, CDCl₃) δ 8.04 (1H, s), 7.47 (1H, s), 7.4] (1H, d, *J*=7.2Hz), 7.27(1H, t, *J*=7.3 Hz), 7.11 (1H, d, *J =* 7.3Hz), 5.16 (2H, s). 3.93 (3H,-s), 3.77 (4H, s), 1.03 (6H,s), *m*/*z* (ES⁺) 302 (M+H⁺).

### Step 3: 3-[3-(1-Methyl-1H-[1,2,4]triazol-3-ylmetboxy)phenyl]-7-trifluoromethylimidazo[1,2-a]pyrimidine

The reaction was carried out as described in Example 1 using 3-bromo-7-trifluoromethylimidazo[7,2-*a*]pyrimidine (300 mg, 1.1 mmol), the foregoing boronate ester (679 mg, 2.2 mmol), and *tetrakis*(triphenylphosphine) palladium(0) (130 mg, 10 mol %) in 2M Na₂CO₃ solution (2.2 mL) and THF (4.5 mL) at 65°C for 3 h. After work up, the residue was purified by column chromatography on silica using 70-100% EtOAc/*iso*-hexane containing 1% MeOH and 1 % Et₃N to give the desired *imidazopyrimidine* (252 mg, 60 %). ¹H NMR (360 MHz, *d*₆-DMSO) δ 9.38 (1H, d, *J=* 7.2 Hz), 8.50 (1H, s), 8.28 (1H, s), 7.58-7.45 (2H, m), 7.32 (1H, d, *J=* 7.8 Hz), 7.15 (1H, dd, *J* = 7.9, 2.1 Hz), 5.18 (2H, s), 3.88 (3H, s). *m*/*z* (ES⁺) 375 (M+H⁺).

### Example 11

### 3-(2-Fluoro-3-trifluoromethylphenyl)-7-trifluoromethylimidazo[1,2-a]pyrimidine

### Step 1: 2-(2-Fluoro-3-trifluoromethylphenyl)-5,5-dimethyl-[1,3,2]dioxaborinane and 2-fluoro-3-trifluoromethylphenylboronic acid

The reaction was carried out according to Example 2, Step 1 using 1-bromo-2-fluoro-3-trifluorolmethylbenzene (1.5 g, 6.2 mmol), *bis*(neopentylglycolato)diborane (1.46 g, 6.5 mmol), KOAc (1.21 g, 12.3 mmol) and Pd(dppf)Cl₂ (252 mg, 5 mol %) in 1,4-dioxane (50 mL) to yield a mixture of the *boronic acid* and the *boronate ester* (1:2, 498 mg, ca. 32 %). Boronic acid:¹H NMR (400 MHz, CDCl₃) δ 8.07-8.02 (1H, m), 7.75-7.69 (1H, m), 7.33-7.28 (1H, m). Boronate ester: ¹H NMR (400 MHz, CDCl₃) δ 7.93-7.86 (1H, m), 7.67-7.61 (1H, m), 7.22-7.17 (1H, m). 3.80 (4H, s), 1.05 (6H,s).

### Step 2:3-(2-Fluoro-3-trifluoromethylphenyl)-7-trifluoromethylimidazo[1,2-a]pyrimidine

The reaction was carried out as described in Example 1 using 3-bromo-7-trifluoromethylimidazo[1,2-*a*]pyrimidine (240 mg, 0.9 mmol), the mixture of the boronate and boronic acid (498 mg, ca. 1.98 mmol), and *tetrakis*(triphenylphosphine)palladium(0) (104 mg, 10 mol %) in 2M Na₂CO₃ solution (1.8 mL) and THF (3.6 mL) at 65°C for 3 h. After work up, the residue was purified by column chromatography on silica using 35% EtOAc/*iso*-hexane to give the desired *imidazopyrimidine* (232 mg, 74 %). ¹H NMR, (400 MHz, *d*₆-DMSO) δ 9.15 (1H, d, *J* = 7.0 Hz), 8.33 (1H, s), 8.09 (1H, t, *J*= 6.6 Hz), 7.97 (1H,t, *J*= 6.3 Hz), 7.68-7.58 (2H, m). m/z (ES⁺) 350 (M+H⁺).

### Example 12

### 3-(3-Trifluoromethoxyphenyl)-7-trifluoromethylimidazo[1,2-a]pyrimidine

The reaction was carried out as described in Example 1 using 3-bromo-7-trifluoromethylimidazo[1,2-*a*]pyrimidine (300 mg, 1.1 mmol), 3-(trifluoromethoxy)benzeneboronic acid (465 mg, 2.2 mmol), and *tetrakis*(triphenylphosphine)palladium(0) (130 mg, 10 mol%) in 2M Na₂CO₃ solution (2.3 mL) and THF (4.5 mL) at 70°C for 90 min. After work up, the residue was purified twice by column chromatography on silica using 30% EtOAc/*iso*-hexane and then 30 % Et₂O/toluene to give the desired *imidazopyrimidine* (160 mg, 42 %). ¹H NMR (360 MHz, *d*₆-DMSO) δ 9.30 (1H, d, *J =* 7.2 Hz), 8.35 (1H, s), 7.84-7.80 (1H, m), 7.81 (1H, s), 7.73 (1H, t, *J =* 8.0 Hz), 7.55 (1H, d, *J* = 7.2 Hz), 7.51 (1H, d, *J =* 8.0 Hz). *m*/*z* (ES⁺) 348 (M+H⁺).

### Example 13

### N-[2-Fluoro-5-(3-trifluoromethylimidazo[1,2-b][1,2,4]triazin-7-yl)phenyl]-N-methyl-N-(3-pyridinylmethyl)amine

The title compound was obtained as an orange solid (18 mg, 94%) using the procedure described in Example 7, Step 2 using N-[2-fluoro-5-(3-trifluoromethylimidazo[1,2*-b*][1,2,4]triazin-7-yl)phenyl]-N-(3-pyridinylmethyl)amine as the amine and paraformaldehyde instead of 3-pyridinecarboxaldehyde. ¹H NMR (400 MHz, d₆-DMSO) δ 2.85 (3H, s), 4.41 (2H, s), 7.34-7.43 (2H, m), 7.70-7.80 (3H, m), 8.46 (1H, dd, *J*= 4.7 and 1.6 Hz), 8.53 (1H, d, *J*= 1-6 Hz), 8.88 (1H, s), 9.30 (1H, s). MS (ES⁺) 403 (M+1).

### Example 14

### 7-[4-Fluoro-3-(pyridin-2-ylethoxy)phenyl]-3-(trifluoromethyl)imidazo[1,2-b][1,2,4]triazine

A mixture of 2-fluoro-5-(3-trifluoromethylimidazo[1,2-*b*][1,2,4]triazin-7-yl)-phenol (60 mg, 0.2 mmol), polymer-supported PPh₃ (133 mg at 3 mmol/g, 0.4 mmol) and 2-(2-hydroxyethyl)pyridine (49 mg, 0.4 mmol) in THF (3 mL) was treated with diethyl azodicarboxylate (69 mg, 0.4 mmol) and stirred at 25°C for 2 h. The reaction mixture was filtered to remove the polymer supported PPh₃ and the filtrate evaporated to give the crude residue- The residue was dissolved in DMSO at 20 mg/mL and a portion (2 mL) of the crude solution was purified by mass-triggered preparative HPLC. The title compound was isolated (18 mg). ¹H NMR (400 MHz, d₆.DMSO) δ 3.28 (2H, t, *J* = 6.6 Hz), 4.56 (2H, t, *J* = 6.6 Hz), 7.23-7.29 (1H, m), 7.38-7.47 (2H, m), 7.53 (1H, dt, *J =* 7.8 and 1.9 Hz), 7.84 (1H, ddd, *J =* 8.4, 4.3 and 2.4 Hz), 7.98 (1H, dd, *J =* 8.4 and 2.4-Hz), 8.51-8.54 (1H, d, *J =* 5.9 Hz), 8.95 (1H, s), 9.35 (1H, s). MS (ES⁺) 404 (M+1).

### Example 15

### 2-Fluoro-5-[3-(trifluoromethyl)imidazo[1,2-b][1,2,4]triazin-7-yl]benzaldehyde

The title compound was isolated as a yellow solid (880 mg, 54%) using the procedure in Example 3, Step 3 using 5-bromo-2-fluorobenzaldehyde instead of 4-bromo-1-fluoro-2-methoxybenzene. ¹H NMR (360 MHz, d₆.DMSO) δ 7.69 (1H, dd, *J* = 10.2 and 8.6 Hz), 8.54 (1H, ddd, *J*= 8.6, 4.9 and 2.4 Hz), 8.71 (1H, dd, *J* = 6.6 and 2.4 Hz), 8.99 (1H, s), 9.42 (1H, s), 10.3 (1H, s). MS (ES⁺) 311 (M+1).

### Example 16

### 3-(3-Trifluoromethylphenyl)imidazo[1,2-a]pyrimidine

3-(3-Trifluoromethylphenyl)imidazo[1,2-*a*]pyrimidine was obtained as a colourless solid (100 mg, 38%) using the procedure described in Example 1, Step 3 using 3-bromoimidazo[1,2-*a*]pyrimidine (WO 01/90108) (0.2 g, 1.0 mmol) and 3-(trifluoromethyl)benzene boronic acid (0.25 g, 1.3 mmol). ¹H NMR (400 MHz, CDCl₃) δ 6.96 (1H, m), 7.66-7.76 (3H, m), 7.81 (1H, s), 7.97 (1H, s), 8.60-8.64 (2H, m). *m*/*z* (ES⁺) 264 (M+H⁺).

### Example 17

### 7-[3-(1H-Benzimidazol-2-ylmethoxy)-4-fluorophenyl]-3-trifluoromethylimidazo[1,2-b][1,2,4]triazine

2-Fluoro-5-(3-trifluoromethylimidazo[1,2-*b*][1,2,4]triazin-7-yl)-phenol (75 mg, 0.25 mmol) and 2-(chloromethyl)benzimidazole hydrochloride (52 mg, 30 mmol) were coupled using the procedure described in Example 10, Step 1 to give the title compound as a yellow solid (23 mg, 21%). ¹H NMR (400 MHz, CDCl₃) δ 5.57 (2H, s), 7.28-7.35 (3H, m), 7.52-7.80 (3H, m), 7.95 (1H, dd *J* = 8.0 and 2.2 Hz), 8.47 (1H, s), 8.52 (1H, s). *m*/*z* (ES⁺) 429 (M+H⁺).

### Example 18

### 7-Chloro-3-(3-trifluoromethylphenyl)imidazo[1,2-a]pyridine

A mixture of 3-bromo-7-chloroimidazo[1,2-*a*]pyridine (WO 01/38326), 3-trifluoromethylbenzeneboronic acid, 2 M Na₂CO₃ solution, and *tetrakis*(triphenylphosphine)palladium(0) in THF were reacted as described in Example 5 to afford the desired *imidazopyridine* (312 mg, 62%). ¹H NMR (400 MHz, d₆-DMSO) δ 7.42 (1H, dd, *J* = 7.4 and 2.3 Hz), 7.87 (1H, t, *J* = 7.8 Hz), 7.95 (1H, d, *J* = 7.8 Hz), 8.04 (1H, d, *J=* 7.4 Hz), 8.08 (1H, s), 8.19 (1H, dd, *J* = 2.3 and 0.8 Hz), 8.39 (1H, s) 8.77 (1H, dd, *J* = 7.4 and 0.8 Hz). *m*/*z* (ES⁺) 297 (MH⁺).

### [Examples 19 to 21 deleted]

### Example 22

### 7-(3-Chlorophenyl)-3-trifluoromethylidazo[1,2-b][1,2,4]triazine

The title compound was made following the procedure of Example 1, Step 3 using 7-bromo-3-trifluoromethylimidazo[1,2-*b*][1,2,4]triazine (3 g, 11.2 mmol), 3-chlorobenzeneboronic acid (3.51 g, 22.5 mmol), 2N Na₂CO₃ solution (22.5 mL), DME (40 mL) and *tetrakis*(triphenylphosphine)palladium(0) (650 mg, 0.6 mmol) to yield the title compound (1.11 g, 33%). ¹HNMR (400 MHz, CDCl₃): δ 7.45-7.52 (2H, m), 7.98 (1H, d, *J*= 7.4 Hz), 8-16 (1H, s), 8.62 (1H, s), 8.88 (1H, s); *m*/*z* (ES+) 299, 301 ratio 3:1 (M+H⁺).

### Example 23

### 3-Trifluoromethyl-7-(3-trifluoromethylsulfanylphenyl)imidazo[1,2-b][1,2,4]triazine

The title compound was made following the procedure of Example 1, Step 3 using 7-bromo-3-trifluoromethylimidazo[1,2-*b*][1,2,4]triazine (0.15 g, 0.56 mmol), 5,5-dimethyl-2-(3-trifluoromethylsulfanylphenyl)-[1,3,2]dioxaborinane (0.24 g, 0.84 mmol), 2N Na₂CO₃ solution (0.84 mL), DME (2 mL) and *tetrakis*(triphenylphosphine)palladium(0) (32 mg, 0.03 mmol) to yield the title compound (87 mg, 43%). ¹HNMR (400 MHz, CDCl₃): δ 7.64 (1H, t, *J=* 7.9 Hz), 7.77 (1H, d, *J* = 7.8 Hz), 8.21 (1H, d, *J* = 7.8 Hz), 8.46 (1H, s), 8.65 (1H, s), 8.86 (1H, s); *m*/*z* (ES+) 365 (M+H⁺).

### Example 24

### 7-[3-(1-Methyl-1H-[1,2,4]triazol-3-ylmethoxy)phenyl]-3-trifluoromethylimidazo[1,2-b][1,2,4]triazine

The reaction was carried out according to Example 1, Step 3 using 7-bromo-3-trifluoromethylimidazo[1,2-*b*][1,2,4]triazine (0.1g, 0.36 mmol), 3-[3-(5,5-dimethyl-[1,3,2]dioxaborinan-2-yl)phenoxymethyl]-1-methyl-1*H-*[1,2,4]triazole (0.14 g, 0.45 mmol), 2N Na₂CO₃ solution (0.45 mL), DME (2 mL) and *tetrakis*(triphenylphosphine)palladium(0) (22 mg, 0.02 mmol) to yield the title compound (83 mg, 59%). ¹HNMR (400 MHz, CDCl₃): δ 3.95 (3H, s), 5.25 (2H, s), 7.16-7.19 (1H, dd, *J=* 8.2, 2.7 Hz), 7.48 (1H, t, *J=* 8.1 Hz), 7.71 (1H, d, *J* = 7.8 Hz), 7-86-7-86 (1H, m), 8.06 (1H, s), 8.61 (1H, s), 8.79 (1H, s); *m*/*z* (ES+) 376 (M+H⁺).

The following compounds can be made by analogous procedures to those disclosed in the foregoing Examples.

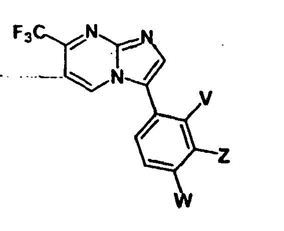

| **V** | **Z** | **W** |
|---|---|---|
| H | NO₂ | F |
| H | NH₂ | F |
| H | Br | F |
| H | 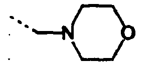 | F |
| H | 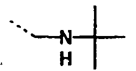 | F |
| H | CH₂OCH₃ | F |
| H | OCH₃ | F |
| H | SCH₃ | H |
| H | OCH₃ | H |
| H | H | F |
| H | N(CH₃)₂ | H |
| H | Cl | H |
| H | Br | H |
| H | 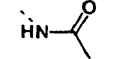 | H |
| H | COCH₃ | H |
| H | 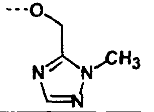 | H |
| H | F | H |
| H | F | F |
| H | 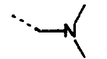 | F |
| H | CH(CH₃)₂ | H |
| H | CH₃ | H |
| H | CN | F |
| H | CF₃ | F |
| H | NO₂ | H |
| H | CON(CH₃)₂ | H |
| H | SO₂CH₃ | H |
| H | 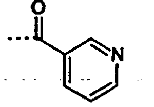 | H |
| H | CF₂H | H |
| H | SCF₃ | H |
| H | SO₂N(CH₃)₂ | H |
| H | CF₃ | Cl |
| H | CF₃ | CH₃ |
| H | 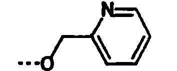 | H |
| H | 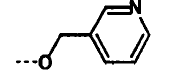 | H |
| H | 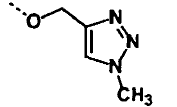 | H |
| F | OCF₃ | H |
| F | CN | H |
| H | 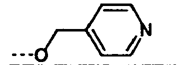 | H |
| H | 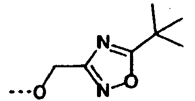 | H |
| H | 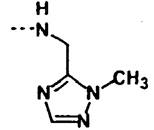 | H |
| H | 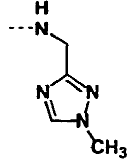 | H |
| H | 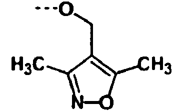 | H |
| H | 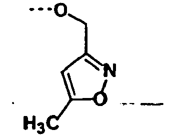 | H |
| H | 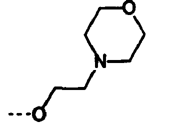 | H |
| H | OCH₂CN | H |
| H | 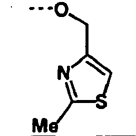 | H |
| H | 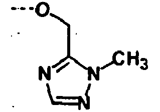 | F |
| H | 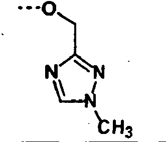 | F |
| H | 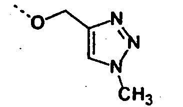 | F |
| Cl | CF₃ | H |

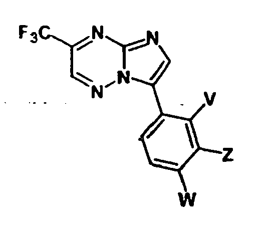

| **V** | **Z** | **W** |
|---|---|---|
| H | 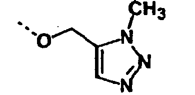 | F |
| H | CH₂Cl | F |
| H | 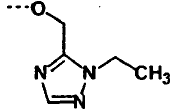 | F |
| H | 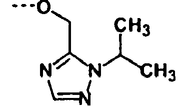 | F |
| H | 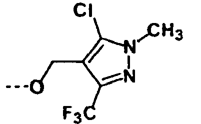 | F |
| H | 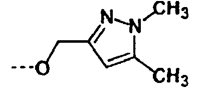 | F |
| H | 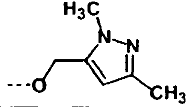 | F |
| H | 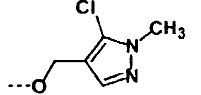 | F |
| H | H | F |
| H | 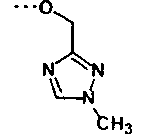 | F |
| H | 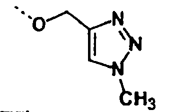 | F |
| H | OEt | F |
| H | OCH₂CF₃ | F |
| H | 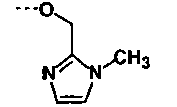 | F |
| H | 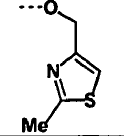 | F |
| H | 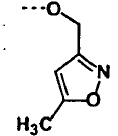 | F |
| H | OCH₂CN | F |
| H | 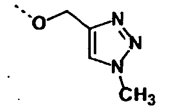 | H |
| H | 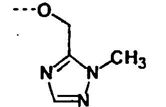 | H |
| H | 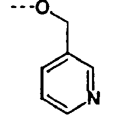 | F |
| H | 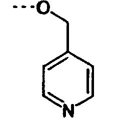 | F |
| H | 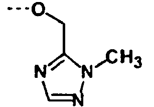 | F |
| H | 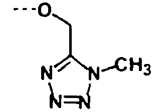 | F |
| H | 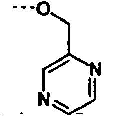 | F |
| H | 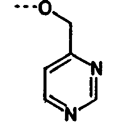 | F |
| H | 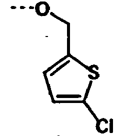 | F |
| H | O¹Pr | F |
| H | 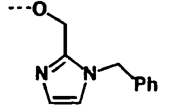 | F |
| H | 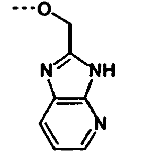 | F |
| H | 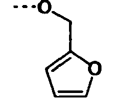 | F |
| H | 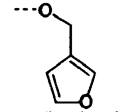 | F |
| H | 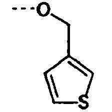 | F |
| H | 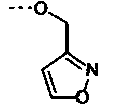 | F |
| H | 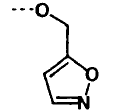 | F |
| H | 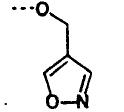 | F |
| H | 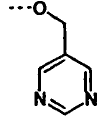 | F |
| H | 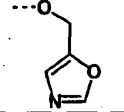 | F |
| H | 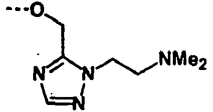 | F |
| H | 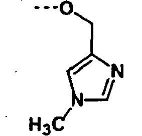 | F |
| H | 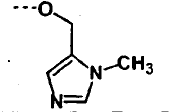 | F |
| H | 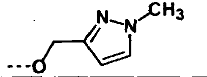 | F |
| H | 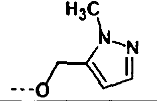 | F |
| H | 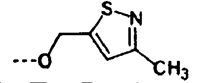 | F |
| H | 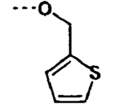 | F |
| H | 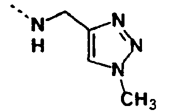 | F |
| H | 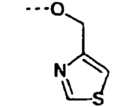 | F |
| H | 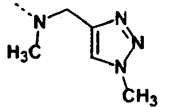 | F |
| H | 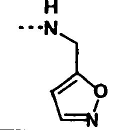 | F |
| H | 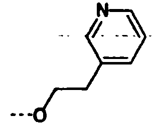 | F |
| H | 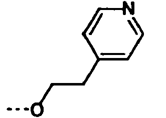 | F |
| H | 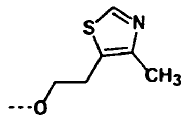 | F |
| H | OCH₂CF₂H | F |
| H | O(CH₂)₂OMe | F |
| H | O(CH₂)₂NMe₂ | F |
| H | 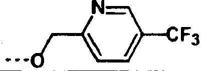 | F |
| H | 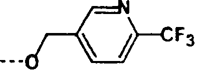 | F |
| H | 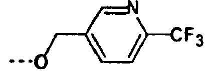 | F |
| H | 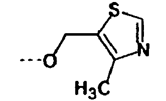 | F |
| H | 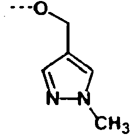 | F |
| H | 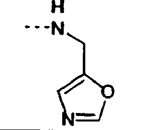 | F |
| H | CH₂OH | F |

## Claims

1. A pharmaceutical composition comprising a compound of formula I or a pharmaceutically acceptable salt thereof: 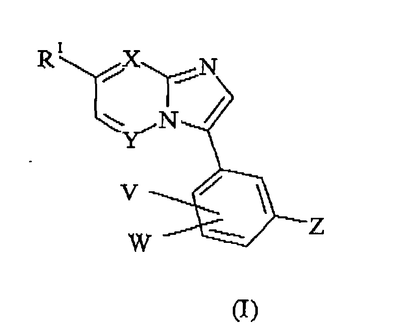 wherein:
X represents N and Y represents N or CH;
R¹ represents hydrogen, hydrocarbon, a heterocyclic group, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b} or -CR^{a}=NOR^{b};
R^{a} and R^{b} independently represent hydrogen, hydrocarbon or a heterocyclic group;
V and W are independently selected from H, halogen, C₁₋₆alkyl, OH and C₁₋₆alkoxy;
Z represents H, halogen, CN, NO₂, CFa, OCF₃, CF₂H, SCF₃, R², OR³, SR³, (CH₂)ₚN(R³)₂, O(CH₂)ₚN(R³)₂, SO₂R², SO₂N(R³)₂, COR⁴, CO₂R³, CON(R³)₂, NHCOR⁴, NR'(CH₂)ₙheteroaryl or O(CH₂)ₙheteroaryl where the heteroaryl is optionally substituted by one, two or three groups chosen from C₁₋₆alkyl, benzyl, (CH₂)ₚN(R³)₂, halogen and CF₃, R' is C₁₋₆alkyl, n is 1 or 2 and p is 0, 1 or 2;
with the proviso that at least one of V, W and Z is other than H;
R² represents C₁₋₆alkyl, C₃₋₆cycloalkyl, C₃₋₆cycloalkylC₁₋₄alkyl, C₂₋₆alkenyl or C₂₋₆alkynyl, any of which may bear a substituent selected from halogen, CN, NO₂ CF₃, OCF₃, CF₂H, SCF₃, OH, C₁₋₄alkoxy, C₁₋₄alkoxycarbonyl, amino, C₁₋₄alkylamino, or di(C₁₋₄alkyl)amino;
R³ represents H or R²; or two R³ groups bonded to the same nitrogen atom may complete a 5-7 membered nonaromatic heterocyclic ring; and
R⁴ represents R³ or heteroaryl;
in a pharmaceutically acceptable carrier.

2. A composition according to claim 1 in which X and Y are CH.

3. A composition according to claim 1 in which X and Y are N.

4. A composition according to claim 1 in which X is N and Y is CH.

5. A composition according to claim 1 in which the compound is:
3-(3-trifluoromethylphenyl)-7-trifluoromethylimidazo[1,2-*a*]pyrimidine;
3- [3-cyanophenyl] -7-trifluoromethylimidazo[1,2-*a*]pyrimidine;
7-{3-[(1,5-dimethyl-1*H*-pyrazol-4-yl)methoxyl-4-fluorophenyl}-3-trifluoromethylimidazo[1,2-*b*] [1,2,4]triazine;
7-[4-fluoro-3-(1-methyl-5-trifluoromethyl-1*H*-pyrazol-4-ylmethoxy)phenyl]-3-trifluoromethylimidazo[1,2-*b*] [1,2,4]triazine;
3-[3-(1-methyl-1*H-*[1,2,4]triazol-3-ylmethoxy)phenyl]-7-trifluoromethylimidazo[1,2-*a*]pyrimidine;
3-(2-fluoro-3-trifluoromethylphenyl)-7-trifluoromethylimidazo[1,2-*a*]pyrimidine;
3-(3-trifluoromethoxyphenyl)-7-trifluoromethylimidazo[1,2-*a*]pyrimidine;
or a pharmaceutically acceptable salt thereof.

6. A composition according to claim 1 in which the compound is:
7-(4-fluoro-3-methoxyphenyl-3-trifluoromethylimidazo[1,2-*b*][1,2,4]triazine;
N-[2-fluoro-5-(3-trifluoromethylimidazo[1,2-*b*][1,2,4]triazin-7-yl)phenyl]-N-methyl-N-(3-pyridinylmethyl)amine
7-[4-fluoro-3-(pyridin-2-ylethoxy)phenyl]-3-(trifluoromethyl)imidazo[1,2-*b*][1,2,4]triazine;
2-fluoro-5-[3-(trifluoromethyl)imidazo[1,2-*b*] [1,2,4]triazin-7-y1]benzaldehyde;
3-(3-trifluoromethylphenyl)imidazo[1,2-*a*]pyrimidine;
7-[3(1*H*-benzimidazol-2-ylmethoxy)-4-fluorophenyl]-3-trifluoromethylimidazo[1,2-*b*][1,2,4]triazine;
7-(3-chlorophenyl)-3-trifluoromethylimidazo[1,2-*b*][1,2,4]triazine;
3-trifluoromethyl-7-(3-trifluoromethylsulfanylphenyl)imidazo[1,2-*b*][1,2,4]triazine;
7-[3-(1-methyl-1*H*-[1,2,4]triazol-3-ylmethoxy)phenyl]-3-trifluoromethylimidazo[1,2-*b*][1,2,4]triazine;
or a pharmaceutically acceptable salt thereof.

7. A pharmaceutical composition comprising a compound selected from:
N-[2-fluoro-5-(3-trifluoromethylimidazo[1,2-*b*][1,2,4]triazin-7-yl)phenyl]-N-(3-pyridinylmethyl)amine;
N-(1,5-dimethyl-1*H*-pyrazol-4-ylmethyl)-N-[2-fluoro-5-(3-trifluoromethylimidazo[1,2-*b*] [1,2,4]triazin-7-yl)phenyl]amine;
N-[3-(7-trifluoromethylimidazo[1,2-*a*]pyrimidin-3-yl)phenyl)]-N-(1-methyl-[1,2,4]triazol-5-ylmethyl)amine;
N-[3-(7-trifluoromethylimidazo[1,2-*a*]pyrimidin-3-yl)phenyl]-N-(2-methyl-[1,2,4]triazol-5-ylmethyl)amine;
N-[2-fluoro-5-(3-trifluoromethylimidazo[1,2,*b*] [1,2,4]triazin-7-yl)phenyl)]-N-(1-methyl-[1,2,3]triazol-4-ylmethyl)amine;
N- [2-fluoro-5-(3-trifluoromethylimidazo[1,2,*b*] [1,2,4]triazin-7-yl)phenyl]-N-(isoxazol-5-ylmethyl)amine; and
N-[2-fluoro-5-(3-trifluoromethylimidazo[1,2,*b*][1,2,4]triazin-7-yl)phenyl]-N-(oxazol-5-ylmethyl)amine;
or a pharmaceutically acceptable salt thereof, in a pharmaceutically acceptable carrier.

8. A compound as defined in anyone of claims 1 to 7 for use in a method of treatment of the human or animal body by therapy.

9. Use of a compound as defined in any one of claims 1 to 7 or a pharmaceutically acceptable salt thereof for the manufacture of a medicament for enhancing cognition.

10. Use of a compound as defined in any one of claims 1 to 7 or a pharmaceutically acceptable salt thereof for the manufacture of a medicament for treating Alzheimer's Disease.

## Patentansprüche

1. Eine pharmazeutische Zusammensetzung, die eine Verbindung der Formel I oder ein pharmazeutisch annehmbares Salz davon: 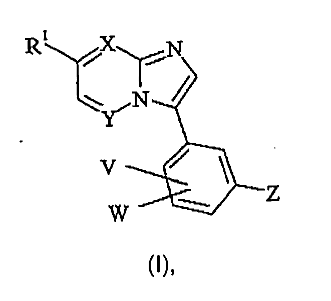 wobei:
X N bedeutet und Y N oder CH bedeutet,
R¹ Wasserstoff, Kohlenwasserstoff, eine heterocyclische Gruppe, Halogen, Cyano, Trifluormethyl, Nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b} oder -CR^{a}=NOR^{b} bedeutet,
R^{a} und R^{b} unabhängig Wasserstoff, Kohlenwasserstoff oder eine heterocyclische Gruppe bedeuten,
V und W unabhängig ausgewählt sind aus H, Halogen, C₁₋₆-Alkyl, OH und C₁₋₆-Alkoxy,
Z H, Halogen, CN, NO₂, CF₃, OCF₃, CF₂H, SCF₃, R², OR³, SR³, (CH₂)ₚN(R³)₂, O(CH₂)ₚN(R³)₂, SO₂R², SO₂N(R³)₂, COR⁴, CO₂R³, CON(R³)₂, NHCOR⁴, NR'(CH₂)ₙ-Heteroaryl oder O(CH₂)ₙ-Heteroaryl bedeutet, wobei das Heteroaryl gegebenenfalls substituiert ist durch ein, zwei oder drei Gruppen, ausgewählt aus C₁₋₆-Alkyl, Benzyl, (CH₂)ₚN(R³)₂, Halogen und CF₃, R' C₁₋₆-Alkyl ist, n 1 oder 2 ist und p 0, 1 oder 2 ist,
mit der Maßgabe, dass wenigstens einer der Reste V, W und Z anders als H ist,
R² C₁₋₆-Alkyl, C₃₋₆-Cycloalkyl, C₃₋₆-Cycloalkyl-C₁₋₄-alkyl, C₂₋₆-Alkenyl oder C₂₋₆-Alkinyl bedeutet, wobei jedes davon einen Substituenten tragen kann, ausgewählt aus Halogen, CN, NO₂, CF₃, OCF₃, CF₂H, SCF₃, OH, C₁₋₄-Alkoxy, C₁₋₄-Alkoxycarbonyl, Amino, C₁₋₄-Alkylamino oder Di(C₁₋₄-alkyl)amino,
R³ H oder R² bedeutet, oder zwei R³-Gruppen, die an dasselbe Stickstoffatom gebunden sind, einen 5-7-gliedrigen nichtaromatischen heterocyclischen Ring vervollständigen können, und
R⁴ R³ oder Heteroaryl bedeutet,
in einem pharmazeutisch annehmbaren Träger enthält.

2. Eine Zusammensetzung gemäß Anspruch 1, wobei X und Y CH sind.

3. Eine Zusammensetzung gemäß Anspruch 1, wobei X und Y N sind.

4. Eine Zusammensetzung gemäß Anspruch 1, wobei X N ist und Y CH ist.

5. Eine Zusammensetzung gemäß Anspruch 1, wobei die Verbindung ist:
3-(3-Trifluormethylphenyl)-7-trifluormethylimidazo[1,2-a]pyrimidin,
3-[3-Cyanophenyl]-7-trifluormethylimidazo[1,2-a]pyrimidin,
7-{3-[(1,5-Dimethyl-1*H*-pyrazol-4-yl)methoxy]-4-fluorphenyl}-3-trifluormethylimidazo-[1,2-b][1,2,4]triazin,
7-[4-Fluor-3-(1-methyl-5-trifluormethyl-1*H*-pyrazol-4-ylmethoxy)phenyl]-3-trifluormethyl-imidazo[1,2-*b*][1,2,4]triazin,
3-[3-(1-Methyl-1*H*-[1,2,4]triazol-3-ylmethoxy)phenyl]-7-trifluormethylimidazo[1,2-*a*]-pyrimidin,
3-(2-Fluor-3-trifluormethylphenyl)-7-trifluormethylimidazo[1,2-a]pyrimidin,
3-(3-Trifluormethoxyphenyl)-7-trifluormethylimidazo[1,2-a]pyrimidin
oder ein pharmazeutisch annehmbares Salz davon.

6. Eine Zusammensetzung gemäß Anspruch 1, wobei die Verbindung ist:
7-(4-Fluor-3-methoxyphenyl-3-trifluormethylimidazo[1,2-*b*][1,2,4]triazin,
N-[2-Fluor-5-(3-trifluormethylimidazo[1,2-*b*][1,2,4]triazin-7-yl)phenyl]-N-methyl-N-(3-pyridinylmethyl)amin,
7-[4-Fluor-3-(pyridin-2-ylethoxy)phenyl]-3-(trifluormethyl)imidazo[1,2-*b*][1,2,4]triazin,
2-Fluor-5-[3-(trifluormethyl)imidazo[1,2-*b*][1,2,4]triazin-7-yl]benzaldehyd,
3-(3-Trifluormethylphenyl)imidazo[1,2-*a*]pyrimidin,
7-[3(1*H*-Benzimidazol-2-ylmethoxy)-4-fluorphenyl]-3-trifluormethylimidazo[1,2-*b*][1,2,4]-triazin,
7-(3-Chlorphenyl)-3-trifluormethylimidazo[1,2-*b*][1,2,4]triazin,
3-Trifluormethyl-7-(3-trifluormethylsulfanylphenyl)imidazo[1,2-*b*][1,2,4]triazin,
7-[3-(1-Methyl-1*H*-[1,2,4]triazol-3-ylmethoxy)phenyl]-3-trifluormethylimidazo[1,2-*b*]-[1,2,4]triazin
oder ein pharmazeutisch annehmbares Salz davon.

7. Eine pharmazeutische Zusammensetzung, enthaltend eine Verbindung, ausgewählt aus:
N-[2-Fluor-5-(3-trifluormethylimidazo[1,2-*b*][1,2,4]triazin-7-yl)phenyl]-N-(3-pyridinylmethyl)amin,
N-(1,5-Dimethyl-1 H-pyrazol-4-ylmethyl)-N-[2-fluor-5-(3-trifluormethylimidazo[1,2-*b*]-[1,2,4]triazin-7-yl)phenyl]amin,
N-[3-(7-Trifluormethylimidazo[1,2-*a*]pyrimidin-3-yl)phenyl)]-N-(1-methyl[1,2,4]triazol-5-ylmethyl)amin,
N-[3-(7-Trifluormethylimidazo[1,2-*a*]pyrimidin-3-yl)phenyl]-N-(2-methyl[1,2,4]triazol-5-ylmethyl)amin,
N-[2-Fluor-5-(3-trifluormethylimidazo[1,2,*b*][1,2,4]triazin-7-yl)phenyl)]-N-(1-methyl-[1,2,3]triazol-4-ylmethyl)amin,
N-[2-Fluor-5-(3-trifluormethylimidazo[1,2,*b*][1,2,4]triazin-7-yl)phenyl]-N-(isoxazol-5-ylmethyl)amin und
N-[2-Fluor-5-(3-trifluormethylimidazo[1,2,*b*][1,2,4]triazin-7-yl)phenyl]-N-(oxazol-5-ylmethyl)amin,
oder ein pharmazeutisch annehmbares Salz davon in einem pharmazeutisch annehmbaren Träger.

8. Eine Verbindung wie in irgendeinem der Ansprüche 1 bis 7 definiert zur Verwendung bei einem Verfahren zur Behandlung des menschlichen oder Tierkörpers durch Therapie.

9. Verwendung einer wie in irgendeinem der Ansprüche 1 bis 7 definierten Verbindung oder eines pharmazeutisch annehmbaren Salzes davon zur Herstellung eines Medikaments zur Kognitionssteigerung.

10. Verwendung einer wie in irgendeinem der Ansprüche 1 bis 7 definierten Verbindung oder eines pharmazeutisch annehmbaren Salzes davon zur Herstellung eines Medikaments zur Behandlung von Alzheimer-Erkrankung.

## Revendications

1. Composition pharmaceutique comprenant un composé de formule I ou un sel pharmaceutiquement acceptable de celui-ci : 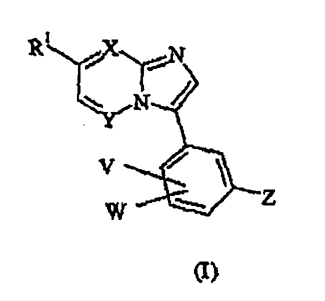 dans laquelle :
X représente N et Y représente N ou CH ;
R¹ représente un atome d'hydrogène, un hydrocarbure, un groupe hétérocyclique, un atome d'halogène, un groupe cyano, trifluorométhyle, nitro, - OR^{a}, -SR^{a}, -SOR^{a}, -SO₂R^{a}, -SO₂NR^{a}R^{b}, -NR^{a}R^{b}, -NR^{a}COR^{b}, - NR^{a}CO₂R^{b}, -COR^{a}, -CO₂R^{a}, -CONR^{a}R^{b} ou -CR^{a}=NOR^{b};
R^{a} et R^{b} représentent indépendamment un atome d'hydrogène, un hydrocarbure ou un groupe hétérocyclique;
V et W sont indépendamment choisis parmi H, un atome d'halogène, un groupe alkyle en C_{1 à 6}, OH et alcoxy en C_{1 à 6} ;
Z représente H, un atome d'halogène, un groupe CN, NO₂, CF₃, OCF₃, CF_{2H}, SCF₃, R², OR³, SR³, (CH₂)ₚN(R³)₂, O(CH₂)ₚN(R³)₂, SO₂R², SO₂N(R³)₂, COR⁴, CO₂R³, CON(R³)₂, NHCOR⁴, NR' (CH₂)ₙhétéroaryle ou O(CH₂)ₙhétéroaryle où l'hétéroaryle est facultativement substitué par un, deux ou trois groupes choisis parmi un groupe alkyle en C_{1 à 6}, benzyle, (CH₂)ₚN(R³)₂, un atome d'halogène et CF₃, R' est un groupe alkyle en C_{1 à 6}, n vaut 1 ou 2 et p vaut 0, 1 ou 2 ;
à la condition qu'au moins l'un de V, W et Z soit autre que H ;
R² représente un groupe alkyle en C_{1 à 6}, cycloalkyle en C_{3 à 6}, cycloalkyl (en C_{3 à 6})-alkyle en C_{1 à 4}, alcényle en C_{2 à 6} ou alcynyle en C_{2 à 6}, dont tous peuvent porter un substituant choisi parmi un atome d'halogène, un groupe CN, NO₂, CF₃, OCF₃, CF₂H, SCF₃, OH, alcoxy en C_{1 à 4}, alcoxycarbonyle en C_{1 à 4}, amino, alkylamino en C_{1 à 4} ou di(alkyl en C_{1 à 4})amino ;
R³ représente H ou R² ; ou deux groupes R³ liés au même atome d'azote peuvent compléter un cycle hétérocyclique non aromatique à 5 à 7 chaînons; et
R⁴ représente R³ ou un groupe hétéroaryle;
dans un support pharmaceutiquement acceptable.

2. Composition selon la revendication 1, dans laquelle X et Y sont CH.

3. Composition selon la revendication 1, dans laquelle X et Y sont N.

4. Composition selon la revendication 1, dans laquelle X est N et Y est CH.

5. Composition selon la revendication 1, dans laquelle le composé est :
la 3-(3-trifluorométhylphényl)-7-trifluorométhylimidazol[1,2-*a*]pyrimidine ;
la 3-[3-cyanophényl]-7-trifluorométhylimidazo[1,2-*a*]pyrimidine ;
la 7-{3-[(1,5-diméthyl-1*H*-pyrazol-4-yl)méthoxy]-4-fluorophényl}-3-trifluorométhylimidazo[1,2-*b*][1,2,4]triazine ;
la 7-[4-fluoro-3-(1-méthyl-5-trifluorométhyl-1*H-*pyrazol-4-ylméthoxy)phényl]-3-trifluorométhylimidazo[1,2-*b*][1,2,4]triazine ;
la 3-[3-(1-méthyl-1*H*-[1,2,4]triazol-3-ylméthoxy)phényl]-7-trifluorométhylimidazo[1,2-*a*]pyrimidine ;
la 3-(2-fluoro-3-trifluorométhylphényl)-7-trifluorométhylimidazo[1,2-*a*]pyrimidine ;
la 3-(3-trifluorométhoxyphényl)-7-trifluorométhylimidazo[1,2-*a*]pyrimidine ;
ou un sel pharmaceutiquement acceptable de celui-ci.

6. Composition selon la revendication 1, dans laquelle le composé est :
la 7-[4-fluoro-3-méthoxyphényl-3-fluorométhylimidazo[1,2-*b*][1,2,4]triazine;
la N-[2-fluoro-5-(3-trifluorométhylimidazo[1,2-*b*][1,2,4]triazin-7-yl)phényl]-N-méthyl-N-(3-pyridinylméthyl)amine ;
la 7-[4-fluoro-3-(pyridin-2-yléthoxy)phényl]-3-(trifluorométhyl)imidazo[1,2-*b*][1,2,4]triazine ;
le 2-fluoro-5-[3-(trifluorométhyl)imidazo[1,2-*b*][1,2,4]triazin-7-yl]benzaldéhyde ;
la 3-(3-trifluorométhylphényl)imidazo[1,2-*a*]pyrimidine ;
la 7-[3-(1*H*-benzimidazol-2-ylméthoxy)-4-fluorophényl]-3-trifluorométhylimidazo[1,2-*b*][1,2,4]triazine ;
la 7-(3-chlorophényl)-3-trifluorométhylimidazo[1,2-*b*][1,2,4]triazine ;
la 3-trifluorométhyl-7-(3-trifluorométhylsulfanylphényl)imidazo[1,2-*b*] [1,2,4]triazine
la 7-[3-(1-méthyl-1*H*-[1,2,4]triazol-3-ylméthoxy)phényl]-3-trifluorométhylimidazo[1,2-*b*][1,2,4]triazine;
ou un sel pharmaceutiquement acceptable de celui-ci.

7. Composition pharmaceutique comprenant un composé choisi parmi :
la N-[2-fluoro-5-(3-trifluorométhylimidazo[1,2-*b*][1,2,4]triazin-7-yl)phényl]-N-(3-pyridinylméthyl)amine ;
la N-(1,5-diméthyl-1*H*-pyrazol-4-ylméthyl)-N-[2-fluoro-5-(3-trifluorométhylimidazo[1,2-*b*][1,2,4]triazin-7-yl)phényl]amine ;
la N-[3-(7-trifluorométhylimidazo[1,2-*a*]pyrimidin-3-yl)phényl)]-N-(1-méthyl-[1,2,4]triazol-5-ylméthyl)amine ;
la N-[3-(7-trifluorométhylimidazo[1,2-*a*]pyrimidin-3-yl)phényl]-N-(2-méthyl-[1,2,4]triazol-5-ylméthyl)amine ;
la N-[2-fluoro-5-(3-trifluorométhylimidazo[1,2-*b*] [1,2,4] triazin-7-yl)phényl)]-N-(1-méthyl-[1,2,3]triazol-4-ylméthyl)amine ;
la N-[2-fluoro-5-(3-trifluorométhylimidazo [1,2-*b*] [1,2,4] triazin-7-yl)phényl]-N-(isoxazol-5-ylméthyl)amine ; et
la N-[2-fluoro-5-(3-trifluorométhylimidazo[1,2-*b*][1,2,4]triazin-7-yl)phényl]-N-(oxazol-5-ylméthyl)amine;
ou un sel pharmaceutiquement acceptable de celui-ci, dans un support pharmaceutiquement acceptable.

8. Composé tel que défini dans l'une quelconque des revendications 1 à 7, pour une utilisation dans un procédé de traitement du corps humain ou d'un animal par thérapie.

9. Utilisation d'un composé tel que défini dans l'une quelconque des revendications 1 à 7 ou d'un sel pharmaceutiquement acceptable de celui-ci, pour la fabrication d'un médicament destiné à améliorer la cognition.

10. Utilisation d'un composé tel que défini dans l'une quelconque des revendications 1 à 7 ou d'un sel pharmaceutiquement acceptable de celui-ci, pour la fabrication d'un médicament destiné à traiter la maladie d'Alzheimer.
